# EUROPÄISCHE PATENTSCHRIFT

(11) **EP 3 164 494 B1**
(45) Veröffentlichungstag und Bekanntmachung des Hinweises auf die Patenterteilung: **02.05.2018**
(21) Anmeldenummer: 15730138.3
(22) Anmeldetag: 17.06.2015
(51) Int. Cl.: C12N 15/68, C12N 15/72, C12N 15/73, C12N 15/90

(54) **T7-EXPRESSIONSSYSTEM, VERFAHREN ZU SEINER HERSTELLUNG UND SEINE VERWENDUNG ZUR HERSTELLUNG VON REKOMBINANTEN PROTEINEN**
T7 EXPRESSIONSYSTEM, METHOD OF PRODUCING IT AND USE THEREOF FOR THE PRODUCTION OF RECOMBINANT PROTEINS
SYSTÈME D'EXPRESSION T7, MÉTHODE POUR SA PRODUCTION, UTILISATION POUR LA PRODUCTION DES PROTÉINES RECOMBINANTES

(30) Priorität: 01.07.2014 DE 102014212675
(43) Veröffentlichungstag der Anmeldung: 10.05.2017
(73) Patentinhaber: Wacker Chemie AG, 81737 München (DE)
(72) Erfinder: ANTON, Andreas, 06120 Halle (DE); JANOWSKI-EGLER, Monique, 06114 Halle (DE); LIEBSCHER, Markus, 06120 Halle (DE)
(74) Vertreter: Potten, Holger
(86) Internationale Anmeldenummer: PCT/EP2015/063575
(87) Internationale Veröffentlichungsnummer: WO 2016/000961

(56) Entgegenhaltungen:
- US-A- 5 824 528
- PEUBEZ ISABELLE ET AL: "Antibiotic-free selection in E. coli: new considerations for optimal design and improved production", MICROBIAL CELL FACTORIES, BIOMED CENTRAL, LONDON, NL, Bd. 9, Nr. 1, 7. September 2010 (2010-09-07), Seite 65, XP021077213, ISSN: 1475-2859, DOI: 10.1186/1475-2859-9-65
- DUBENDORFF J W ET AL: "Creation of a T7 autogene - Cloning and expression of the gene for bacteriophage T7 RNA polymerase under control of its cognate promoter", JOURNAL OF MOLECULAR BIOLOGY, ACADEMIC PRESS, UNITED KINGDOM, Bd. 219, Nr. 1, 5. Mai 1991 (1991-05-05), Seiten 61-68, XP024021411, ISSN: 0022-2836, DOI: 10.1016/0022-2836(91)90857-3 [gefunden am 1991-05-05]
- DATSENKO KIRILL A ET AL: "One-step inactivation of chromosomal genes in Escherichia coli K-12 using PCR products", PROCEEDINGS OF THE NATIONAL ACADEMY OF SCIENCES, NATIONAL ACADEMY OF SCIENCES, US, Bd. 97, Nr. 12, 6. Juni 2000 (2000-06-06), Seiten 6640-6645, XP002210218, ISSN: 0027-8424, DOI: 10.1073/PNAS.120163297
- DE MOERLOOZE L ET AL: "Stabilization of T7-promoter-based pARHS expression vectors using the parB locus", GENE, ELSEVIER, AMSTERDAM, NL, Bd. 119, Nr. 1, 21. September 1992 (1992-09-21), Seiten 91-93, XP023540003, ISSN: 0378-1119, DOI: 10.1016/0378-1119(92)90070-6 [gefunden am 1992-09-21]
- WALIA RUPALI ET AL: "Development of expression vectors for Escherichia coli based on the pCR2 replicon", MICROBIAL CELL FACTORIES, BIOMED CENTRAL, LONDON, NL, Bd. 6, Nr. 1, 10. Mai 2007 (2007-05-10), Seite 14, XP021024081, ISSN: 1475-2859, DOI: 10.1186/1475-2859-6-14
- FIEDLER M ET AL: "proBA complementation of an auxotrophic E. coli strain improves plasmid stability and expression yield during fermenter production of a recombinant antibody fragment", GENE, ELSEVIER, AMSTERDAM, NL, Bd. 274, Nr. 1-2, 22. August 2001 (2001-08-22), Seiten 111-118, XP004319600, ISSN: 0378-1119, DOI: 10.1016/S0378-1119(01)00629-1

## Beschreibung

Die Erfindung betrifft ein T7 Expressionssystem, Verfahren zu seiner Herstellung und seine Verwendung zur antibiotikafreien Herstellung von rekombinanten Proteinen.

T7-Expressionsysteme werden für die Expression rekombinanter Proteine verwendet. In der Regel befindet sich auf einem Vektor die T7-Promotorsequenz und downstream davon nach einer Ribosomen-Bindungsstelle die kodierende Region für ein rekombinates Protein. Da vom Expressionsorganismus keine T7-Polymerase produziert wird, ist ein solches Expressionssystem nur funktionsfähig, wenn die T7-Polymerase künstlich in das System eingebracht wurde. Die häufigste Methode hierzu ist die genomische Verankerung einer T7-Expressionskassette im Produktionsorganismus. Seit langem ist ein T7-Polymerase basiertes prokaryontisches System für die Expression von Proteinen in BL21 (DE3) *Escherichia coli* Zellen bekannt. In diesem System erfolgt die Integration der T7-Polymerase in das *E. coli* Genom mit Hilfe des DE3 Lambda Phagens in die Lambda-Attachment site von *E. coli.* In dem System erkennt die T7-Phagen-Polymerase den T7-Phagen-Promoter. Dieses System hat jedoch den Nachteil, dass die Plasmidstabilität bei Zielgen-Expression gering ist. Zahlreiche Studien haben versucht, die Expressionslevel des T7-Expressionssystems zu stabilisieren, jedoch bislang ohne nennenswerten Erfolg. Zudem enthält *E. coli* ca. 40 kb Lambda-Phagen-DNA. Diese Phagen-DNA ist zumindest in Systemen für die Expression von Pharmaproteinen unerwünscht. In T7-Expressions-systemen werden bisher zudem immer Expressionsvektoren eingesetzt, die ein Antibiotika-Resistenzgen als Selektivmarker für die Klonierung und Selektion während der Expression enthalten.

Der Einsatz von Antibiotika zur Selektion von Plasmiden ist jedoch ein entscheidender Nachteil bei der Herstellung insbesondere therapeutischer Proteine. Ein antibiotikafreies Verfahren findet eine größere Akzeptanz seitens der Regulierungsbehörden, da das Produkt sicherer für den Patienten sein wird und man aufgrund der geringen Endproduktanalytik (Abreicherung des Antibiotikums im Produkt) einen deutlich preiswerteren Prozess konstruieren kann. Ein Expressionsvektor, der ein Verfahren zur antibiotikafreien Herstellung von Proteinen ermöglicht, ist beispielsweise aus EP1697523B1 bekannt. Dieser Expressionsvektor ist allerdings nicht für ein T7-Expressionssystem geeignet.

Aufgabe der vorliegenden Erfindung ist es, ein T7-Expressionssystem für die antibiotikafreie Produktion eines rekombinanten Proteins bestehend aus einer prokaryontischen Zelle und einem Expressionsvektor zur Verfügung zu stellen, welches eine im Vergleich zu bekannten T7-Expressionssystemen erhöhte Stabilität des Expressionsvektors aufweist.

Diese Aufgabe wird gelöst durch ein Expressionssystem umfassend eine prokaryontische Zelle enthaltend ein Nukleotidkonstrukt kodierend für eine T7-RNA-Polymerase unter Kontrolle eines T7-Promotors und unter Kontrolle eines induzierbaren Promotors, sowie einen Expressionsvektor, der ein Gen kodierend ein zu exprimierendes rekombinantes Protein unter der Kontrolle eines T7-Promotors enthält, dadurch gekennzeichnet, dass die prokaryontische Zelle keine Lambda-Phagen-DNA enthält und die T7-RNA-Polymerase unter Kontrolle eines T7-Promotors und unter Kontrolle eines induzierbaren Promotors mittels einer "site specific" Rekombination in das Genom der prokaryontischen Zelle gezielt integriert wurde und der Expressionsvektor ein Plasmidstabilisierungssystem aufweist.

Plasmidstabilisierungssysteme sind beispielsweise aus Sengupta and Austin, Infection and Immunity, July 2011, p. 2502-2509 bekannt. Vorzugsweise handelt es sich um ein Plasmidstabilisierungssystem aus der Gruppe multimer resolution Systeme (mrs), partitioning Systeme (par) und postsegregational killing Systeme (PSK). Besonders bevorzugt weist das Plasmidstabilisierungs-system eine Sequenz aus der Gruppe der multimer resolution Systeme auf, insbesondere bevorzugt handelt es sich um die cer-Sequenz.

Bei dem induzierbaren Promotor handelt es sich vorzugsweise um einen Promotor aus der Gruppe tac, lac, trp, phoA. Besonders bevorzugt handelt es sich um den induzierbaren Promotor lacUV5.

Bei dem Nukleotidkonstrukt handelt es sich bevorzugt um ein Nukleotidkonstrukt bestehend aus der Nukleotidsequenz kodierend für die T7-RNA-Polymerase, der Nukleotidsequenz des lacUV5-Promotors inklusive des 5'-Endes des *lacZ*-Gens aus *E. coli* und der T7-Promotor Sequenz. Ein derartiges Nukleotidkonstrukt ist in SEQ ID NO:1 wiedergegeben. Das Nukleotidkonstrukt ist insbesondere bevorzugt ins Genom der prokaryontischen Zelle des erfindungsgemäßen Expressionssystems integriert.

Bei der prokaryontischen Zelle handelt es sich vorzugsweise um eine Zelle eines *E.* coli-Stammes mit dem T7-RNA-Polymerasegen im Genom. Derartige Stämme lassen sich in an sich bekannter Weise aus Wildtyp-Stämmen wie sie in Stammsammlungen erhältlich sind, herstellen. Beispiele für derartige Stämme sind die *E. coli*-Stämme BL21 (käuflich erhältlich bei Stratagene, La Jolla USA) HMS174 (DSM5932), oder C600 (DSM426) (erhältlich unter den genannten DSM Nummern bei der Deutschen Sammlung für Mikroorganismen und Zellkulturen GmbH (DSMZ), 38124 Brauschweig, Inhoffenstraße 7B).

Vorzugsweise wird das erfindungsgemäße T7-Expressionssystem hergestellt, indem in diese Stämme ein im Rahmen der vorliegenden Erfindung entwickelter T7-Expressionsvektor eingebracht wird.

Ein derartiger Expressionsvektor weist vorzugsweise folgende Bestandteile in funktioneller Verbindung auf:
einen Replikationsursprung (ori),
einen T7-Promotor als regulatorische Sequenz,
eine Sequenz, die für ein zu exprimierendes rekombinantes Protein kodiert
ein Antibiotika-Resistenzgen als Selektivmarker für die Klonierung,
einen Terminator,
ein Repressorgen,
eine für das Repressorgen geeignete Operatorsequenz,
ein Plasmidstabilisierungsystem.

Vorzugsweise handelt es sich um eines der bereits genannten Plasmidstabilisierungsysteme. Besonders bevorzugt weist das Plasmidstabilisierungsystem die cer-Sequenz auf.

Vorzugsweise handelt es sich bei dem Replikationsursprung um den pBR322-ori, den pUC19-ori oder den p15A-ori, besonders bevorzugt um den pBR322-ori.

Besonders bevorzugt handelt es sich bei dem Terminator um t₀, rrnB oder den T7-Terminator.

Ein Repressorgen, ist jedes Gen, das ein Protein kodiert, das bei Bindung im Promotorbereich die Transkription von Genen verhindert.Besonders bevorzugt handelt es sich bei dem Repressorgen um das *lacI*-Gen.

Vorzugsweise handelt es sich bei dem Antibiotika-Resistenzgen um kan, tet, bla, cm^{R}, tetA besonders bevorzugt um kan.

Das erfindungsgemäße T7-Expressionssystems lässt sich herstellen wie bereits bekannte T7-Expressionssysteme, mit dem Unterschied, dass man einen beschreibenen Expressionsvektor in eine prokaryontischen Zelle mit einem T7-RNA-Polymerasegen im Genom einbringt.

Der Vorteil der vorliegenden Erfindung ist außerdem die Nutzung in antibiotikafreien Fermentationen in Komplexmedium, wodurch in der Regel eine höhere Expressionsleistung des herzustellenden Proteins erzielt werden kann, als bei der Verwendung von Mineralsalzmedium, wie bei dem in EP1697523B1 beschriebenen Expressionssystem. Die Erfindung betrifft somit auch ein Verfahren zur Produktion eines rekombinanten Proteins mittels eines erfindungsgemäßen Expressionssystems.

Dieses Verfahren ist dadurch gekennzeichnet, dass ein erfindungsgemäßes Expressionssystem unter Antibiotika-freien Bedingungen, die für die Expression der T7-RNA-Polymerase und die Expression des rekombinanten Proteins ausgehend von einer Polynukleotidsequenz geeignet sind, fermentiert werden, wobei das rekombinante Polypeptid exprimiert wird und das rekombinante Polypeptid isoliert wird.

Bei der vorliegenden Erfindung erfolgt der Fermentationsprozess, d.h. der eigentliche Verfahrensabschnitt zur Expression der Proteine, in einer vollkommen antibiotikafreien Umgebung. Dadurch können die eingangs genannten Probleme, die mit dem Einsatz von Antibiotika zur Selektion von Plasmiden verbunden sind, beispielsweise Vorbehalte der Regulierungsbehörden, Produktsicherheit, Endproduktanalytik (Abreicherung des Antibiotikums im Produkt) und die damit verbundenen Risiken und Kosten, umgangen werden.

Unter Proteinen sind im Sinne der vorliegenden Erfindung auch Peptide und Polypeptide zu verstehen.

Das erfindungsgemäße Verfahren wird vorzugsweise in einem Bioreaktor durchgeführt.
Fig. 1 zeigt schematisch das T7-Nukleotidkonstrukt im Genom von BL21 (DE3) (Stand der Technik) und BL21 Δ*proBA*-T7lacZ (erfindungsgemäß) im Vergleich;
   a) DE3: Integration in lambda-attachment-site mit Hilfe eines veränderten lambda-Phagens (Stand der Technik)
   b) Scil-T7: gezielte Integration durch gleichzeitige Deletion von *proBA* (erfindungsgemäß)
Fig. 2 zeigt schematisch das T7-Nukleotidkonstrukt bestehend aus *lacl, lac*UV5-Promotor, 5'-Region von *lacZ,* T7-Promotor und *gene 1* des T7-Phagens (T7-Polymerase) sowie des von zwei FRT sites flankierten Kanamycinresistenzgens *kan*
Fig. 3 zeigt schematisch den Expressionsvektors pSCIL006c bestehend aus T7-Promotor, *t0*-Terminator, pBR322-ori, *proBA, kan* und *lacl.*
Fig. 4 zeigt schematisch die Expressionsvektoren pSCIL122 und pSCIL123 bestehend aus T7-Promotor, *t0*-Terminator, pBR322-ori, *proBA, kan, lacI* und cer.
Fig. 5 zeigt schematisch die Expressionsvektoren pSCIL124 und pSCIL125 bestehend aus T7-Promotor, *t0*-Terminator, pBR322-ori, *proBA, tetA, lacI* und cer.
Fig 6. zeigt schematisch den Expressionsvektor pSCIL129 bestehend aus T7-Promotor, *t0*-Terminator, pBR322-ori, *kan, lacI* und cer

Die folgenden Beispiele dienen zur weiteren Erläuterung der Erfindung.

### Beispiel 1: Herstellung von Expressionsvektoren und -plasmiden

### Herstellung des Expressionsplamids pSCIL006c

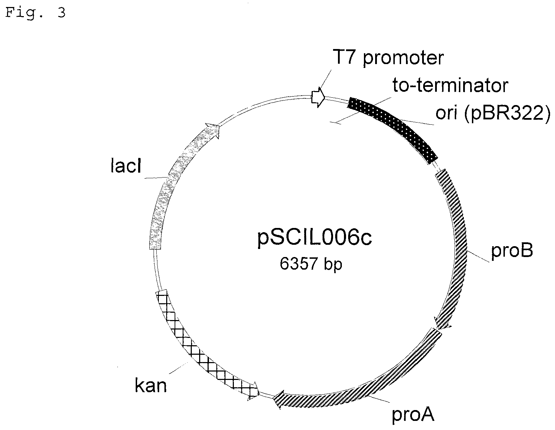

Die Herstellung des Expressionsvektors pSCIL006c (Fig. 3) wird im Folgenden genauer beschrieben.

### 1. Insertion des t0-Terminators in pUC19 = pSCIL001

Der kommerziell erhältliche Vektor pUC19 (SD0061, MBI Fermentas) wurde als Grundlage für die Herstellung der Expressionsvektoren benutzt. pUC19 wurde mit den Endonukleasen HindIII und *Afl*III geschnitten, was zur Deletion von 359 bp der Vektorsequenz führte. Der *t0*-Transkriptionsterminator wurde von DNA des Phagens Lambda (SD0011, MBI Fermentas) mit Hilfe der Primer t0-OD-MCS-HindIII (SEQ ID NO: 2) und t0-UU-MCS-AflIII (SEQ ID NO: 3) amplifiziert. Das entstandene Fragment wurde anschließend mit den Endonukleasen *Hind*III und *Afl*III geschnitten und nach Aufreinigung (Minielute Kit, Qiagen) in das PUC19^{*Hind*III/*Afl*III}-Fragment kloniert. Das resultierende Plasmid pSCIL101 wurde mittels Restriktionsanalysen und Sequenzierung überprüft.

### 2. Austausch der Antibiotikaresistenzkassette in pSCIL001 = pSCIL002

Die Kanamycinresistenzkassette wurde aus pACYC177 (E4151 S, NEB) mittels PCR mit den Primern Km-OD-ApaI (SEQ ID NO: 4) und Km-UU-NheI (SEQ ID NO: 5) amplifiziert. Das Fragment wurde mit tels Gel Extraction Kit (Qiagen) gereinigt und in pGEM-T easy (A1360, Promega) kloniert. Das Plasmid pSCIL101 wurde ohne die Ampicillinresistenzkassette mit Hilfe der Primer pUC2541-OD-NheI (SEQ ID NO: 6) und pUC1496-UU-ApaI (SEQ ID NO: 7) amplifiziert und mit den Endonukleasen ApaI und *Nhe*I geschnitten. Die Kanamycinresistenzkassette wurde aus pGEMT-T easy mit den Endonukleasen *Apa*I und *NheI* herausgeschnitten und in pSCIL001 (ΔAmp)^{*Apa*I/*Nhe*I} kloniert. Das resultierende Plasmid pSCIL002 wurde mittels Restriktionsanalysen überprüft.

### 3. Änderung der Orientierung der Kanamycinresistenzkassette = pSCIL002b

Die Orientierung der Kanamycinresitenzkassette in pSCL-Vektoren spielt eine entscheidende Rolle bei der Hintergrundexpression des Kanamycinresistenzgens, deshalb wurde die Orientierung der Resistenzkassette in der zweiten Vektor-Generation im Vergleich zu z.B. pSCIL008 (EP1697523B1) geändert. Dafür wurde der Backbone von pSCIL002 zunächst ohne Kanamycinresitenzkassette mit Hilfe der Primer MunI-Km term (SEQ ID NO: 8) und ApaI-pSCIL002 (SEQ ID NO: 9) amplifiziert und anschließend mit den Nukleasen *Mun*I (*Mfe*I) und ApaI geschnitten. Die Kanamycinresistenzkassette wurde mit den Primern Km 5'-MunI (SEQ ID NO: 10) und Km 3' ApaI (SEQ ID NO: 11) aus pSCIL002 amplifiziert und ebenfalls mit den Endonukleasen Mun*I* (*Mfe*I) und ApaI geschnitten. Beide Fragmente wurden anschließend ligiert, was zum Plasmid pSCIL002b führte. Die korrekte Integration wurde durch Sequenzierung überprüft.

### 4. Insertion eines zweiten Selektionsmarkers in pSCIL002b = pSCIL003b

Die Gene für den Prolinsyntheseweg von *E. coli, proBA,* wurden anschließend als zweiter Selektionsmarker in pSCIL002b integriert. Dazu wurde zunächst das *proBA*-Operon mit Hilfe der Primer proBA-OD-ApaI (SEQ ID NO: 12) und proBA-UU-ApaI (SEQ ID NO: 13) aus zuvor präparierter genomischer DNA von *E. coli* K12 (DSM 9037, Deutschen Sammlung für Mikroorganismen und Zellkulturen GmbH; DNeasy Tissue Kit, Qiagen) amplifiziert. Das PCR-Produkt wurde mit der Endonuklease ApaI geschnitten und in den ebenfalls mit *Apa*I geschnittenen Vektor pSCIL002b kloniert. Das resultierende Plasmid pSCIL003b wurde durch Sequenzierung überprüft.

### 5. Insertion des Repressorgens lacI in pSCIL003b = pSCIL004b

Das *lacI* Gen inklusive der nativen Promotorregion wurde von chromosomaler DNA des *E. coli*-Stammes K12 (DSM 9037, Deutschen Sammlung für Mikroorganismen und Zellkulturen GmbH; DNeasy Tissue Kit, Qiagen) mit Hilfe der Primer lacI OD NheI (SEQ ID NO: 14) und lacI UU NheI (SEQ ID NO: 15) amplifiziert. Das PCR-Produkt wurde in pGEM-T easy (A1360, Promega) kloniert und durch Sequenzierung überprüft. Nach Restriktion mit der Endonuklease *Nhe*I wurde *lacI* in pSCIL003b ligiert.

### 6. Insertion des T7-Promotors in pSCIL004b = pSCIL006b

Der T7-Promotor inklusive der *lac*-Operatorsequenz wurde vom Plasmid pSCIL006 (original aus pTYB2, #6710, NEB) mit Hilfe der Primer PrT7-MunI5'(SEQ ID NO: 16) und PrT7-EcoRI3'(SEQ ID NO: 17) amplifiziert. Durch den Primer PrT7-EcoRI3' wurde außerdem die spätere Ribosomenbindestelle (AGGAGA) in das PCR-Produkt integriert. Das PCR-Produkt wurde mit den Endonukleasen EcoRI und MunI (*Mfe*I) geschnitten und in den mit EcoRI-geschnittenen Vektor pSCIL004b kloniert. Das resultierende Plasmid pSCIL006b wurde durch Sequenzierung überprüft.

### 7. Änderung des Replikationsursprungs (origin of replication) durch Austausch eines Nukleotids in pSCIL006b = pSCIL006c

Das Expressionsplasmid pSCIL006b zeigt als Abkömmling von pUC19 bei 37 °C eine sehr viel höhere Kopienzahl als bei 30 °C. Der Grund dafür ist eine Punktmutation (G→A) im origin of replication des Originalplasmids pBR322. Um diese Mutation in pSCIL006b rückgängig zu machen, wurde der Phusion Site Directed Mutagenesis Kit (F-541, Finnzymes) genutzt. Dazu wurde das gesamte Plasmid mit den Primern ori_mut_fwd (SEQ ID NO: 18) und ori_rev (SEQ ID NO: 19) amplifiziert und religiert. Das resultierende Plasmid mit einer Kopienzahl, die unabhängig von der Temperatur bei ca. 60 Kopien/Zelle liegt, wurde mittels Sequenzierung überprüft und als pSCIL006c bezeichnet (siehe Fig.3, SEQ ID NO: 20).

### Herstellung der Expressionsvektoren pSCIL122 und pSCIL123

### 1. Insertion von cer in pSCIL006c = pSCIL122 bzw. pSCIL123

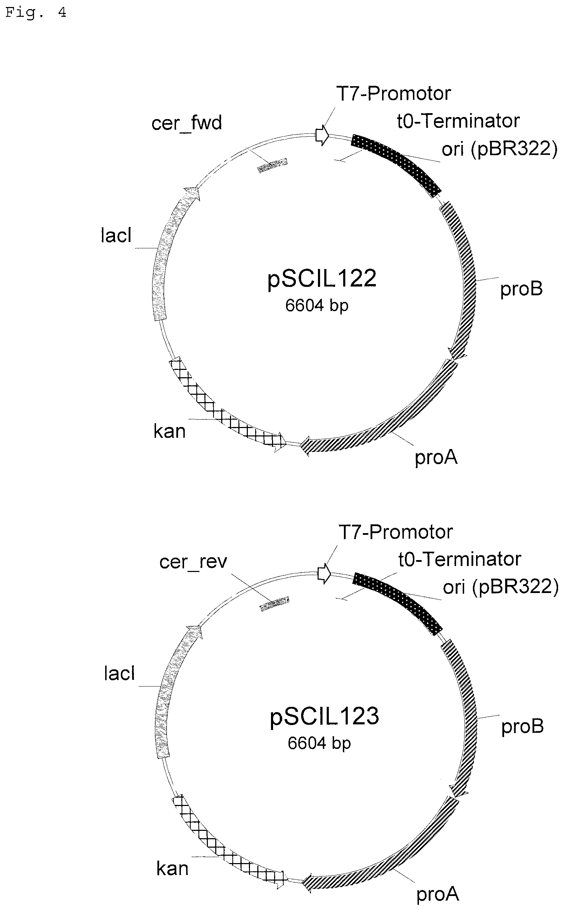

Das cer-Element wurde mittels PCR mit den Primern 5'cer_NdeI (SEQ ID NO: 21) und 3'cer_NdeI (SEQ ID No: 22) aus dem Plasmid ColE1 (isoliert aus *E. coli* JC411 (DSM3877), DSMZ, mittels QIAprep Spin Miniprep, Qiagen) amplifiziert und in eine unique NdeI-Schnittstelle in pSCIL006c integriert. Dabei sind beide Orientierungen (forward und reverse) möglich. Das resultierende Plasmid mit cer in forward Orientierung wurde als pSCIL122, das Plasmid mit cer in reverse Orientierung wurde als pSCIL123 bezeichnet und mittels Sequenzierung überprüft (siehe Fig.4, SEQ ID NO: 23).

### Herstellung der Expressionsvektoren pSCIL124 und pSCIL125

### 1. Deletion von kan in pSCIL006c = pSCIL112

Für die Deletion der Kanamycinresistenzkassette wurde der Phusion Site Directed Mutagenesis Kit (F-541, Finnzymes) benutzt. Dazu wurde der Backbone von pSCIL006c mit den Primern pSCIL_Nco_P (SEQ ID No: 24) und pSCIL_Spe_P (SEQ ID No: 25) amplifiziert und relegiert, was zu Deletion der Kanamycinresistenzkassette führte. Das resultierende Plasmid wurde durch Sequenzierung überprüft und als pSCIL112 bezeichnet.

### 2. Insertion von tetA in pSCIL112 = pSCIL105

Die Tetracyclinresistenzkassette wurde mit den Primern te-tA_5'promo_NcoI (SEQ ID No: 26) und tetA_3'_SpeI (SEQ ID No: 27) von pBR322 (D1511, Promega) amplifiziert. Das PCR-Produkt wurde mit den Endonukleasen NcoI und *Spe*I geschnitten und in den ebenfalls mit *Nco*I und *Spe*I geschnittenen Vektor pSCIL112 kloniert. Da das resultierende Plasmid zwei Kopien statt einer Kopie der Tetracyclinresistenzkassette aufwies, wurde eine Kopie mittels Phusion Site Directed Mutagenesis Kit (F-541, Finnzymes) entfernt. Dazu wurde das Plasmid mit den Primern 105_fwd_P (SEQ ID No: 28) und 105_rev_P (SEQ ID No: 29) amplifiziert und religiert. Das resultierende Plasmid wurde mittels Sequenzierung überprüft und als pSCIL105 bezeichnet.

### 3. Insertion von cer in pSCIL105 = pSCIL124 bzw. pSCIL125

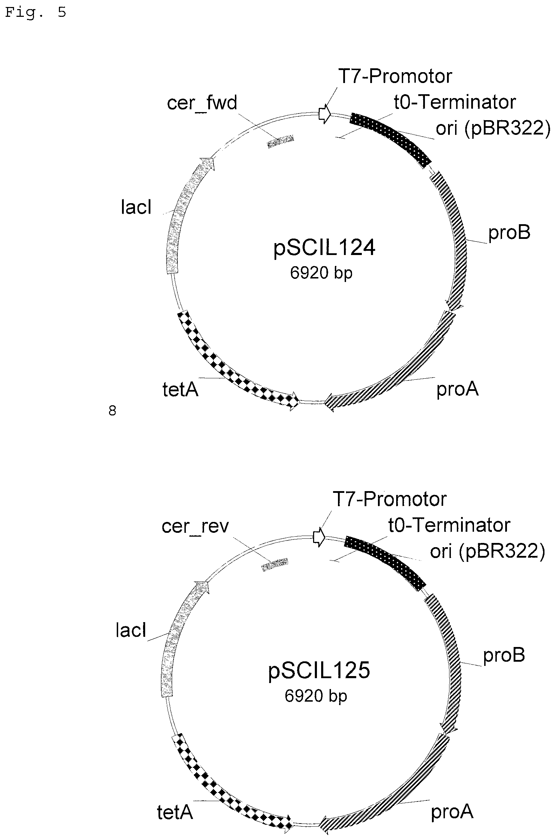

Das cer-Element wurde mittels PCR mit den Primern 5'cer_NdeI (SEQ ID NO: 21) und 3'cer_NdeI (SEQ ID No: 22) aus dem Plasmid ColE1 (isoliert aus *E. coli* JC411 (DSM3877), DSMZ, mittels QIAprep Spin Miniprep, Qiagen) amplifiziert und in eine unique *Nde*I-Schnittstelle in pSCIL105 integriert. Dabei sind beide Orientierungen (forward und reverse) möglich. Das resultierende Plasmid mit cer in forward Orientierung wurde als pSCIL124, das Plasmid mit cer in reverse Orientierung wurde als pSCIL125 bezeichnet und mittels Sequenzierung überprüft (siehe Fig.5, SEQ ID NO: 30).

### Herstellung des Expressionvektors pSCIL129

### 1. Deletion von proBA in pSCIL123 = pSCIL129

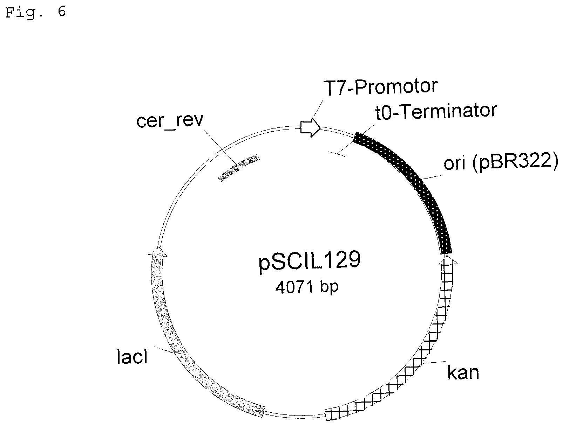

Um die Plasmidgröße des Expressionsvektors zu verringern, wurde die *proBA*-Kassette wieder entfernt. Dazu wurde der Phusion Site Directed Mutagenesis Kit (F-541, Finnzymes) benutzt. Das Plasmid pSCIL123 wurde mit Hilfe der Primer pSCIL-proBA_rev-P (SEQ ID NO: 31) und pSCIL-proBA_fwd-P (SEQ ID NO:32) so amplifiziert, dass nach anschließender Religation das proBA-Operon deletiert wurde. Der resultierende Expressionsvektor wurde pSCIL129 genannt und durch Sequenzierung überprüft (siehe Fig.6, SEQ ID NO: 33).

### Beispiel 2 Herstellung eines erfindungsgemäßen Expressionssystems

Die *E. coli* Stämme BL21 (Stratagene), HMS174 (DSM5932) und C600' (DSM426) wurden von Stratagene bzw. der DSMZ (Deutsche Stammsammlung für Mikroorganismen und Zellkulturen GmbH, Braunschweig) bezogen. Die Stämme BL21, HMS174 und C600' wurden jeweils mit dem Plasmid pKD46 (CGSC, E. coli Genetic Stock Center, Yale Univerity, New Haven, USA), das für die Red-Rekombinase des Lambda-Phagens kodiert, transformiert. Dadurch ist eine zielgerichtete Integration des T7-Konstruktes ins E. *coli*-Genom möglich. Von den resultierenden Stämmen BL21 pKD46, HMS174 pKD46 und C600' pKD46 wurden wiederum kompetente Zellen hergestellt. Die Selektion von pKD46 erfolgte mit Ampicillin auf Agarplatten bzw. Carbenicillin in Flüssiganzuchten.

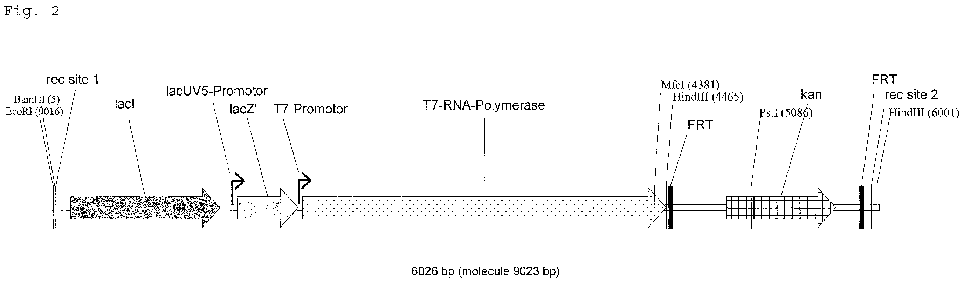

Das in Fig. 2 dargestellte DNA-Konstrukt bestehend aus *lacI, lac*UV5-Promotor, 5'-Region von *lacZ,* T7-Promotor und *gene 1* des T7-Phagens (T7-Polymerase) sowie des von zwei FRT sites flankierten Kanamycinresistenzgens *kan* wurde wie folgt hergestellt. Zunächst wurden die Komponenten einzeln amplifiziert und in pGEM-T easy (A1360, Promega) zusammen kloniert. Das Gen für die T7-RNA-Polymerase (*gene 1*) wurde mit den Primern T7 5'_BamHI_Xho (SEQ ID NO: 34) und T7 3'_Hind (SEQ ID NO: 35) von DNA des T7-Phagens (310005, Bioron GmbH) amplifiziert. Das PCR-Produkt wurde mittels Qiaquick PCR Purification (Qiagen) gereinigt und durch überhängende Adeninreste in den Vektor pGEM-T easy (A1360, Promega) kloniert. Das entstandene Plasmid pGEM::T7 wurde durch Sequenzierung überprüft. Das *lacI*-Gen wurde aus genomischer DNA des *E. coli*-Stammes K12 (DSM 9037, Deutschen Sammlung für Mikroorganismen und Zellkulturen GmbH; DNeasy Tissue Kit, Qiagen) mit Hilfe der Primer lacI 5'_BamHI_coli (SEQ ID NO: 36) und lacI 3'_UV5_Xho (SEQ ID NO: 37) amplifiziert. Die Sequenz des *lac*UV5-Promotors wurde dabei mit Hilfe des Primers lacI 3'_UV5_Xho (SEQ ID NO:37) in das PCR-Produkt integriert. Das PCR-Produkt wurde mittels Qiaquick PCR Purification (Qiagen) gereinigt und durch überhängende Adeninreste in den Vektor pGEM-T easy (A1360, Promega) kloniert. Das entstandene Plasmid pGEM::lacI-lacUV5 wurde durch Sequenzierung überprüft und anschließend mit den Endonukleasen *Bam*HI und *Xho*I geschnitten. Das entstandene Fragment wurde mittels QIAquick Gel Extraction Kit (Qiagen) gereinigt und in das ebenso geschnittene und gereinigte Plasmid pGEM::T7 kloniert.

Das entstandene Plasmid pGEM::lacI-lacUV5-T7 wurde durch Sequenzierung überprüft. Das von FRT sites flankierte Kanamycinresistenzgen wurde mit den Primern Hind_pKD4 5'(SEQ ID NO: 38) und pKD4 3'_Hind_coli (SEQ ID NO: 39) aus dem Plasmid pKD4 (CGSC, E. coli Genetic Stock Center, Yale Univerity, New Haven, USA) amplifiziert. Das PCR-Produkt wurde mit der Endonuklease *Hind*III geschnitten und in das ebenfalls mit *Hind*III geschnittene Plasmid pGEM::lacI-lacUV5-T7 kloniert. Das finale Plasmid pGEM-T easy::lacI-lacUV5-T7-kan-FRT wurde mittels Restriktionsverdau mit den Endonukleasen *Eco*RI bzw. *Hind*III überprüft.

Um ein T7-Nukleotidkonstrukt analog zu DE3 herzustellen, wurde zunächst die Region bestehend aus *lacI, lac*UV5-Promoter, 5'-Region von *lacZ* und der ersten 2574 bp des *gene 1* des T7-Phagens (bis zu einer internen *Mfe*I-Schnittstelle) mit Hilfe der Primer T7_neu_BamHI_for01 (SEQ ID NO: 40) und T7_neu_*Mfe*I_rev02 (SEQ ID NO: 41) aus zuvor isolierter genomischer DNA (Genomic DNA Purification Kit, Fermentas) von BL21 (DE3) [Novagen 69387-3] amplifiziert. Diese PCR-Produkt wurde nach Reinigung (Wizard® SV Gel and PCR, Promega) mit den Endonukleasen *Bam*HI und *Mfe*I geschnitten und nach Aufreinigung (Wizard® SV Gel and PCR, Promega) in das 4647 bp-Fragment des Plasmids pGEM-T easy: : lacI-lacUV5-T7-kan-FRT^{*Bam*HI/*Mfe*I} kloniert. Nach Transformation in TOP10F' (Life Technologies) und Selektion auf LB-Platten mit 100 µg/ml Ampicillin wurde das resultierende Plasmid pGEM-T easy::lacI-lacUV5_lacZ_T7-kan (SEQ ID NO: 42) präpariert.

Anschließend wurde das gesamte Konstrukt aus dem Plasmid pGEM-T easy::lacI-lacUV5_lacZ_T7-kan (SEQ ID NO: 42) mittels der Endonuklease *Eco*RI herausgeschnitten. Das so entstandene 6026 bp-Fragment wurde mittel Wizard® SV Gel and PCR-Kit (Promega) aufgereinigt. Die Stämme BL21 pKD46, HMS174 pKD46 und C600' pKD46 wurden anschließend mit diesem DNA-Fragment transformiert und über Nacht bei Raumtemperatur inkubiert, um eine Integration ins *E*. *coli*-Genom zu ermöglichen.

Durch die Wahl der flankierenden Regionen (SEQ ID NO: 43: rec site 1, SEQ ID NO: 44: rec site 2) des DNA-Konstruktes passend zu den flankierenden Regionen des *proBA*-Operon, wird bei der Integration gleichzeitig das *proBA*-Operon deletiert. Die Selektion auf das in Fig. 2 dargestellte T7-RNA-Polymerase-Konstrukt erfolgte mittels Kanamycin im Medium. Der Verlust des Helferplasmids pKD46 wurde durch den Verlust der Fähigkeit auf Ampicillin- bzw. Carbenicillin-haltigen Medien zu wachsen nachgewiesen.

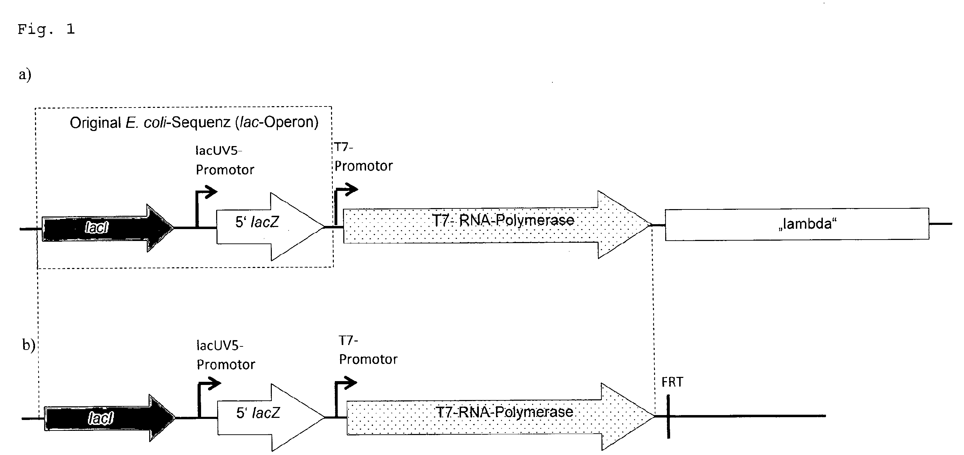

Von den resultierenden Stämmen BL21 Δ*proBA*-T7lacZ::*kan*, HMS174 *ΔproBA*-T7lacZ::*kan* bzw. C600'Δ*proBA*-T7lacZ::*kan* wurden kompetente Zellen hergestellt und mit dem Helferplasmid pCP20 (CGSC, E. coli Genetic Stock Center, Yale Univerity, New Haven, USA) transformiert. Dieser Schritt dient der Deletion der Kanamycin-Resistenz, die im ersten Schritt mit ins Genom integriert wurde, durch die auf pCP20 kodierte FLP-Rekombinase. Die Selektion auf Stämme mit pCP20 erfolgte auf Ampicillin-haltigen Platten. Durch eine Inkubation der Zellen über Nacht bei 43 °C geht das Helferplasmid pCP20 verloren. Die resultierenden Stämme BL21 Δ*proBA*-T7lacZ, HMS174 Δ*proBA*-T7lacZ bzw. C600'Δ*proBA*-T7lacZ besitzen keine Antibiotika-Resistenzen und enthalten das T7-Konstrukt wie in Fig. 1 dargestellt anstelle des proBA-Operons im Genom. Die eine FRT site, die als "Narbe" der Methode im Genom zurückbleibt, entsteht durch die Deletion der KanamycinResistenzkassette durch die FLP-Rekombinase. Die Stämme wurden mittels Southern-Hybridisierung verifiziert. Dabei wurde die Integration der kodierenden Sequenz für die T7-RNA-Polymerase und die Deletion von *proBA* nachgewiesen.

In die so erhaltenen Stämme BL21 Δ*proBA*-T7lacZ und HMS174 Δ*pro-*BA-T7lacZ wurde das Expressionsplasmid aus Beispiel 1 über Elektroporation eingebracht.

### Beispiel 3. Vergleich der Plasmidstabilität eines erfindungsgemäßen T7-Expressionssystems und eines T7-Expressionssystems gemäß Stand der Technik.

Um die Stabilität von T7-Expressionsplasmiden gemäß Stand der Technik und erfindungsgemäß einsetzbaren T7-Expressionsplasmiden in BL21 (DE3) (Stamm aus dem Stand der Technk) und BL21 Δ*proBA*-T7lacZ (für die Erfindung geeigneter Stamm) zu untersuchen, wurden Experimente in Schüttelkolben und Fed-batch-Fermentationen durchgeführt. Dazu wurde das Gen für den humanen Wachstumsfaktor proNGF ausgehend von einer Gensynthese (Geneart, Regensburg) mit Hilfe der Endonukleasen *Ec*oRI und *Pst*I in die Expressionsvektoren kloniert. Für die Untersuchung der Plasmidstabilität im Schüttelkolben wurde wie folgt vorgegangen. Zunächst wurden Vorkulturen der zu untersuchenden Expressionsstämme BL21 (DE3) mit Standardexpressionsplasmid pSCIL006cΔ*proBA*::proNGF (Stand der Technik), BL21 (DE3) mit stabilem Expressionsplasmid pSCIL129::proNGF (erfindungsgemäß ein setzbares Expressionsplasmid), BL21 Δ*proBA*-T7lacZ mit Standardexpressionsplasmid pSCIL006cΔ*proBA*::proNGF (für die Erfindung einesetzbarer Expressionsstamm mit Expressionsplasmid ohne Plasmidstabilisierungssystem) und BL21 Δ*proBA*-T7lacZ mit stabilem Expressionsplasmid pSCIL129::proNGF (erfindungsgemäßes Expressionssystem) in Komplexmedium [34,5 g/l Hefeextrakt (Merck 1.03753.0500), 2,475 mM MgSO₄, 9,347 mM NH₄Cl, 62 mM K₂HPO₄, 15 g/l Glucose] mit 50 µg/ml Kanamycin über Nacht bei 37 °C schüttelnd angezogen. Am nächsten Morgen wurden die Zellen, die notwendig sind, um eine Hauptkultur auf eine Optische Dichte OD₆₀₀ = 0,2-0,3 zu inokulieren, mittels Zentrifugation mit 6500 rpm (5 min) steril geerntet. Die damit inokulierte Hauptkultur (20 ml Komplexmedium ohne Antibiotikum in 100 ml Ehrlenmeyerkolben) wurde bei 37 °C mit 210 rpm schüttelnd inkubiert bis eine OD₆₀₀ = 0,8 erreicht wurde. Zu diesem Zeitpunkt wurde die Zielgenexpression durch Zugabe von 1 mM IPTG induziert. Drei Stunden (3 h pi (post induction)) und 24 h nach Zugabe des Induktors (24 h pi) wurden sterile Verdünnung der Kulturen hergestellt und auf LB-Medium [5 g/l Hefextrakt (Merck 1.03753.0500), 10 g/l Soja-Peptone (Merck 1.07212.0500), 85,56 mM NaCl] ausplattiert. Diese Platten wurden über Nacht bei 37 °C inkubiert. Von den gewachsenen Kolonien wurden jeweils 100 Kolonien parallel auf LB und LB + 50 µg/ml Kanamycin gepickt. Eine Plasmidstabilität von z.B. 10 % bedeutet also, dass nur 10 % der auf LB gewachsenen Kolonien auch auf LB + Kanamycin gewachsen sind. Die Kolonien, die auf LB + Kanamycin wachsen können, besitzen noch die Expressionsplasmide. Kolonien, die nur auf LB wachsen können, haben die Expressionsplasmide verloren. Es wurden jeweils vier unabhängige Experimente durchgeführt, um Aussagen zu den Plasmidstabilitäten zu treffen. Die Ergebnisse sind als Mittelwerte mit Standardabweichungen in Tab. 1 dargestellt.

**Tab. 1: Plasmidstabilität eines Standardexpressionsplasmids gemäß Stand der Technik (pSCIL006cΔproBA::proNGF) und eines erfindungsgemäß einsetzbaren Expressionsplasmids (pSCIL129::proNGF) in BL21 (DE3) (Stamm gemäß Stand der Technik) und BL21 ΔproBA-T7lacZ (für die Erfindung geeigneter Stamm) im Schüttelkolben**

| Zeit punkt | BL21 (DE3) | | BL21 Δ*proBA*-T7lacZ | |
|---|---|---|---|---|
| | | | | |
| | pSCIL006cΔproBA ::proNGF | pSCIL129: :proNGF | pSCIL006cΔpro BA: :proNGF | pSCIL129: :pro NGF |
| 3 h pi | 86 % ± 4 | 100 % | 90 % ± 7 | 100 % |
| 24 h pi | 5 % ± 6 | 88 % ± 6 | 84 % ± 18 | 99 % ± 1 |

Es zeigte sich, dass das Expressionssystem nach Stand der Technik sehr geringe Plasmidstabilitäten von 86 % 3 h nach Induktion und nur 5 % 24 h nach Induktion aufweist. Durch die Stabilisierung des Expressionsplasmides mittels cer gemäß Erfindung ist in BL21 (DE3) eine Steigerung der Plasmidstabilät auf 100 % 3 h nach Induktion und 88 % 24 h nach Induktion möglich. Eine Steigerung der Plasmidstabilität auf ca. 100 % 24 h nach Induktion konnte nur durch die Nutzung eines T7-Expressionssystems gemäß Erfindung, bestehend aus BL21 Δ*proBA*-T7lacZ mit stabilem Expressionsplasmid pSCIL129::proNGF, erreicht werden. Überraschenderweise zeigte aber auch ein Standardexpressionsplasmid in BL21 Δ*proBA*-T7lacZ eine hohe Plasmidstabiliät von 90 % 3 h nach Induktion bzw. 84 % 24 h nach Induktion, wohingegen das gleiche Plasmid in BL21 (DE3) zum gleichen Zeitpunkt bereits weitgehend verloren gegangen war (5% 24 h pi). Als Zielgen wurde in diesen Versuchen der humane Wachstumsfaktor proNGF exprimiert. Die Expression von proNGF wurde mittels SDS-PAGE überprüft.

Das erfindungsgemäße Expressionssystem wurde außerdem in Fed-batch-Fermentationen mit dem Stand der Technik verglichen. Zunächst wurden Vorkulturen der zu untersuchenden Expressionsstämme BL21 (DE3) mit Standardexpressionsplasmid pSCIL006cΔ*proBA*::proNGF (Stand der Technik), BL21 (DE3) mit stabilem Expressionsplasmid pSCIL129::proNGF (erfindungsgemäß einsetzbares Expressionsplasmid), BL21 Δ*proBA*-T7lacZ mit Standardexpressionsplasmid pSCIL006cΔ*proBA*::proNGF (für die Erfindung geeigneter Expressionsstamm mit Expressionsplasmid ohne Plasmidstabilisierungssystem) und BL21 Δ*proBA*-T7lacZ mit stabilem Expressionsplasmid pSCIL129::proNGF (erfindungsgemäßes Expressionssystem) in Komplexmedium [34,5 g/l Hefeextrakt (Merck 1.03753.0500), 2,475 mM MgSO₄, 9,347 mM NH₄Cl, 62 mM K₂HPO₄, 15 g/l Glucose] mit 50 µg/ml Kanamycin über Nacht bei 37 °C schüttelnd angezogen. Für die Fermentation wurden je 2 l Komplexmedium ohne Antibiotikum [50 g/l Hefeextrakt (Biospringer 0206), 2,475 mM MgSO₄, 9,347 mM NH₄Cl, 62 mM K₂HPO₄, 10 g/l Glucose, 0,2 ml/l Antischaum Synperonic (Croda ETK0879)] in 5 1-Laborfermentern vorgelegt und mit Vorkulturen der Expressionsstämme so inokuliert, dass eine Start-OD₆₀₀ (Optische Dichte bei 600 nm) von 0,06 erreicht wurde. Nach 6,5 h, nachdem die Glucose aufgebraucht war, wurde die Fed-batch-Phase durch Zugabe von Substrat gestartet. Die Dosierung des Substrats (feed flow) erfolgt dabei nach einer exponentiellen Funktion der Form Feedflow = const*exp(µ₁*t). Wobei µ die spezifische Wachstumsrate ist, für die µ₁ = const < µₘₐₓ gilt, d. h., dass die momentane zur Verfügung stehende Glucosekonzentration stets unter dem maximalen, momentanen Bedarf der Mikroorganismen liegt, wodurch eine submaximale Wachstumsrate erzielt und eine Akkumulation von Glucose verhindert wird. Es wird z. B. µ₁ = 0,25 h⁻¹ gesetzt. Bei einer definierten Optischen Dichte, z. B. OD₆₀₀ = 50 ± 5, wird die Proteinexpression durch die Zugabe von IPTG, z. B. 1 mM, induziert. Zu diesem Zeitpunkt wird die Dosierung des Substrates (feed flow) linear über 6 h um 70% reduziert und anschließend konstant gehalten. Der Prozess endet nach einer definierten Induktionsdauer, z.B. 24 h.

Bei Erreichen einer OD₆₀₀ = 50 ± 5 wurde die Zielgenexpression durch Zugabe von 1 mM IPTG induziert. Zu diesem Zeitpunkt und 6 h, 8 h und 24 h nach Induktion wurden die Plasmidstabilitäten wie oben beschrieben analysiert und die Expression des humanen Wachstumsfaktor proNGF überprüft. Die Daten sind in Tab. 2 dargestellt.

**Tab. 2 Plasmidstabilität eines Standardexpressionsplasmids (pSCIL006cΔproBA::proNGF) und eines erfindungsgemäß einsetzbaren Expressionsplasmids (pSCIL129::proNGF) in BL21 (DE3) und BL21 ΔproBA-T7lacZ in Fed-batch-Fermentationen**

| Zeitpunkt | | BL21 (DE3) | | BL21 Δ*proBA*-T7lacZ | |
|---|---|---|---|---|---|
| | | | | | |
| | | pSCIL006c ΔproBA: :proNGF | pSCIL129: :proNGF | pSCIL006c ΔproBA: :proNGF | pSCIL129: :proNGF |
| Induktion | OD₆₀₀ | 51,6 | 52,2 | 46,1 | 47,2 |
| | PS | **100%** | 100 % | 100 % | **100 %** |
| 6 h pi | OD₆₀₀ | 97,4 | 101,2 | 99,0 | 106,9 |
| | PS | **11 %** | 100 % | 94 % | **100 %** |
| 8 h pi | OD₆₀₀ | 97,4 | 103,6 | 101,6 | 108,9 |
| | PS | **13 %** | 100 % | 89 % | **100 %** |
| 24 h pi | OD₆₀₀ | 106,6 | 98,8 | 113,6 | 122,9 |
| | PS | **2 %** | 100 % | 95 % | **100 %** |

| | | | | | |
|---|---|---|---|---|---|
| (PS: Plasmidstabilität) | | | | | |

Es zeigte sich, dass das Expressionssystem nach Stand der Technik in einer Fed-batch-Fermentation bereits 6 h nach Induktion 89 % der Expressionsplasmide verloren hat (PS 11%), wohingegen das erfindungsgemäße Expressionssystem auch bis 24 h nach Induktion eine Plasmidstabilität von 100 % aufweist. Dabei hat sowohl das erfindungsgemäß einestzbare Expressionsplasmid mit Plasmidstabilisierungssystem cer (pSCIL129::proNGF) als auch der erfindungsgemäß insetzbare Expressionstamm BL21 Δ*proBA-*T7lacZ klare Vorteile gegenüber dem Expressionssystem nach Stand der Technik, da die Plasmidstabilitäten 24 h nach Induktion hierbei mit 100 % bzw. 95 % signifikant höher liegen als im Expressionssystem nach Stand der Technik mit 2 %.

Um die Stabilität von erfindungsgemäß einestzbaren T7-Expressionsplasmiden in BL21 (DE3) und BL21 Δ*proBA*-T7lacZ weiter zu untersuchen, wurden weitere Experimente in Schüttelkolben durchgeführt. Dazu wurde das Gen für humanes preThrombin ausgehend von einer Gensynthese (Geneart, Regensburg) mit Hilfe der Endonukleasen *Eco*RI und *Pst*I in die Expressionsvektoren pSCIL006c (usual expression plasmid) und pSCIL123 (stable expression plasmid) kloniert. Für die Untersuchung der Plasmidstabilität im Schüttelkolben wurde wie oben beschrieben vorgegangen. Die Ergebnisse sind in Tab. 3 dargestellt.

**Tab. 3: Plasmidstabilität eines Standardexpressionsplasmids (pSCIL006c::preThrombin) und eines erfindungsgemäß einestzbaren Expressionsplasmids (pSCIL123::preThrombin) in BL21 (DE3) und BL21 ΔproBA-T7lacZ**

| Zeitpunkt | BL21 (DE3) | | BL21 Δ*proBA*-T7lacZ | |
|---|---|---|---|---|
| | | | | |
| | pSCIL006c: :preThrombin | pSCIL123: :preThrombin | pSCIL006c: :preThrombin | pSCIL123: :preThrombin |
| 3 h pi | 5 % ± 5 | **100 %** | 6 % ± 3 | **100 %** |
| 24 h pi | 1 % ± 0 | 41 % ± 1 | 1 % ± 2 | **78 % ± 5** |

Es zeigte sich, dass das Expressionssystem nach Stand der Technik extrem geringe Plasmidstabilitäten aufwies. Bereits 3 h nach Induktion waren nur noch 5 % plasmidtragende Stämme vorhanden und nach 24 h nahezu alle Zellen plasmidfrei.

Durch die Verwendung des erfindungsgemäß einsetzbaren Expressionsplasmids pSCIL123::preThrombin konnte in beiden Expressionsstämmen BL21 (DE3) und BL21 Δ*proBA*-T7lacZ 3 h nach Induktion eine Plasmidstabilität von 100 % erreicht werden. Zum Zeitpunkt 24 h nach Induktion ist die Plasmidstabilität des erfindungsgemäßen Expressionssystems (BL21 Δ*proBA*-T7lacZ mit pSCIL123::preThrombin) mit 78 % ca. doppelt so hoch wie im Expressionsstamm nach Stand der Technik (BL21 (DE3) pSCIL123::preThrombin) mit 41 %. Die Expression des humanen preThrombins wurde mittels SDS-PAGE überprüft.

## Patentansprüche

1. Expressionsystem umfassend eine prokaryontische Zelle enthaltend ein Nukleotidkonstrukt kodierend für eine T7 RNA-Polymerase unter Kontrolle eines T7 Promotors und unter Kontrolle eines induzierbaren Promotors, sowie einen Expressionsvektor, der ein Gen kodierend ein zu exprimierendes Protein unter der Kontrolle eines T7 Promotors enthält, **dadurch gekennzeichnet, dass** die prokaryontische Zelle keine Lambda-Phagen-DNA enthält und die T7-RNA-Polymerase unter Kontrolle eines T7-Promotors und unter Kontrolle eines induzierbaren Promotors mittels einer "site specific" Rekombination in das Genom der prokaryontischen Zelle gezielt integriert wurde und der Expressionsvektor ein Plasmidstabilisierungssystem aufweist.

2. Expressionssystem gemäß Anspruch 1, **dadurch gekennzeichnet, dass** das Plasmidstabilisierungssystem ausgewählt ist aus der Gruppe multimer resolution Systeme (mrs), partitioning Systeme (par) und postsegregational killing Systeme (PSK).

3. Expressionssystem gemäß Anspruch 1 oder 2, **dadurch gekennzeichnet, dass** die prokaryontische Zelle die Zelle eines *Escherichia coli* Stammes mit einem T7-RNA-Polymerasegen im Genom ist.

4. Expressionssystem gemäß einem der Ansprüche 1 bis 3, welches ein Nukleotidkonstrukt mit SEQ ID NO: 1 umfasst.

5. Expressionssystem gemäß einem der Ansprüche 3 oder 4, **dadurch gekennzeichnet, dass** die gezielte Integration der T7-RNA-Polymerase unter Kontrolle eines induzierbaren Promotors in die *proBA*-Sequenz von *E. coli* erfolgt.

6. Verfahren zur Produktion eines rekombinanten Proteins mittels eines Expressionssystems gemäß Anspruch 1 bis 5 **dadurch gekennzeichnet, dass** das Expressionssystem unter Antibiotika-freien Bedingungen fermentiert wird, die für die Expression der T7-RNA-Polymerase und die Expression des rekombinanten Proteins ausgehend von einer Polynukleotidsequenz geeignet sind,
wobei das rekombinante Polypeptid exprimiert wird und das rekombinante Polypeptid isoliert wird.

## Claims

1. Expression system comprising a prokaryotic cell containing a nucleotide construct coding for a T7 RNA polymerase under control of a T7 promoter and under control of an inducible promoter, and an expression vector, which contains a gene encoding a protein to be expressed under the control of a T7 promoter, **characterized in that** the prokaryotic cell contains no lambda phage DNA and the T7 RNA polymerase under control of a T7 promoter and under control of an inducible promoter was specifically integrated into the genome of the prokaryotic cell by means of a "site specific" recombination and the expression vector contains a plasmid stabilization system.

2. Expression system according to Claim 1, **characterized in that** the plasmid stabilization system is selected from the group multimer resolution systems (mrs), partitioning systems (par) and postsegregational killing systems (PSK).

3. Expression system according to Claim 1 or 2, **characterized in that** the prokaryotic cell is the cell of an *Escherichia coli* strain with a T7 RNA polymerase gene in the genome.

4. Expression system according to one of Claims 1 to 3, which comprises a nucleotide construct with SEQ ID NO: 1.

5. Expression system according to one of Claims 3 or 4, **characterized in that** the targeted integration of the T7 RNA polymerase under control of an inducible promoter is effected into the *proBA* sequence of *E. coli.*

6. Method for producing a recombinant protein by means of an expression system according to Claim 1 to 5 **characterized in that** the expression system is fermented under antibiotic-free conditions which are suitable for the expression of the T7 RNA polymerase and the expression of the recombinant protein starting from a polynucleotide sequence,
whereby the recombinant polypeptide is expressed and the recombinant polypeptide is isolated.

## Revendications

1. Système d'expression comprenant une cellule procaryote contenant une construction nucléotidique codant pour une ARN-polymérase de T7 régulée par un promoteur de T7 et régulée par un promoteur inductible ainsi que vecteur d'expression qui contient un gène codant pour une protéine à exprimer régulée par un promoteur de T7, **caractérisé en ce que** la cellule procaryote ne contient pas d'ADN de phage lambda et l'ARN-polymérase de T7 régulée par un promoteur de T7 et régulée par un promoteur inductible ayant été intégrée dans le génome de la cellule procaryote au moyen d'une recombinaison spécifique au site ("site specific") et le vecteur d'expression présentant un système de stabilisation plasmidique.

2. Système d'expression selon la revendication 1, **caractérisé en ce que** le système de stabilisation plasmidique est choisi dans le groupe formé par les systèmes de résolution de multimères (multimer resolution systems - mrs), les systèmes de partition (partitioning systems - par) et les systèmes de "post-segregational killing" (PSK).

3. Système d'expression selon la revendication 1 ou 2, **caractérisé en ce que** la cellule procaryote est la cellule d'une souche d'Escherichia coli présentant un gène d'ARN-polymérase de T7 dans le génome.

4. Système d'expression selon l'une quelconque des revendications 1 à 3, qui comprend une construction nucléotidique présentant la séquence SEQ ID NO : 1.

5. Système d'expression selon l'une quelconque des revendications 3 ou 4, **caractérisé en ce que** l'intégration ciblée de l'ARN-polymérase de T7 a lieu par régulation par un promoteur inductible dans la séquence proBA d'E. coli.

6. Procédé pour la production d'une protéine recombinante au moyen d'un système d'expression selon les revendications 1 à 5, **caractérisé en ce que** le système d'expression est fermenté dans des conditions exemptes d'antibiotiques, qui conviennent pour l'expression de l'ARN-polymérase de T7 et pour l'expression de la protéine recombinante partant d'une séquence polynucléotidique, le polypeptide recombinant étant exprimé et le polypeptide recombinant étant isolé.


### SEQUENCE LISTING

<110> Wacker Chemie AG
<120> T7 Expressionssystem, Verfahren zu seiner Herstellung und seine Verwendung zur antibiotikafreien Herstellung von rekombinanten Proteinen
<130> Wa11363F
<160> 44
<170> PatentIn version 3.5
<210> 1
   <211> 5990
   <212> DNA
   <213> Artificial Sequence
<220>
   <223> chromosomales T7-RNA-Polymerasekonstrukt
<400> 1
   acagtcctgc taaaacgttc gtttgatatc atttttcctg acaccatcga atggcgcaaa      60
   acctttcgcg gtatggcatg atagcgcccg gaagagagtc aattcagggt ggtgaatgtg     120
   aaaccagtaa cgttatacga tgtcgcagag tatgccggtg tctcttatca gaccgtttcc     180
   cgcgtggtga accaggccag ccacgtttct gcgaaaacgc gggaaaaagt ggaagcggcg     240
   atggcggagc tgaattacat tcccaaccgc gtggcacaac aactggcggg caaacagtcg     300
   ttgctgattg gcgttgccac ctccagtctg gccctgcacg cgccgtcgca aattgtcgcg     360
   gcgattaaat ctcgcgccga tcaactgggt gccagcgtgg tggtgtcgat ggtagaacga     420
   agcggcgtcg aagcctgtaa agcggcggtg cacaatcttc tcgcgcaacg cgtcagtggg     480
   ctgatcatta actatccgct ggatgaccag gatgccattg ctgtggaagc tgcctgcact     540
   aatgttccgg cgttatttct tgatgtctct gaccagacac ccatcaacag tattattttc     600
   tcccatgaag acggtacgcg actgggcgtg gagcatctgg tcgcattggg tcaccagcaa     660
   atcgcgctgt tagcgggccc attaagttct gtctcggcgc gtctgcgtct ggctggctgg     720
   cataaatatc tcactcgcaa tcaaattcag ccgatagcgg aacgggaagg cgactggagt     780
   gccatgtccg gttttcaaca aaccatgcaa atgctgaatg agggcatcgt tcccactgcg     840
   atgctggttg ccaacgatca gatggcgctg ggcgcaatgc gcgccattac cgagtccggg     900
   ctgcgcgttg gtgcggatat ctcggtagtg ggatacgacg ataccgaaga cagctcatgt     960
   tatatcccgc cgttaaccac catcaaacag gattttcgcc tgctggggca aaccagcgtg    1020
   gaccgcttgc tgcaactctc tcagggccag gcggtgaagg gcaatcagct gttgcccgtc    1080
   tcactggtga aaagaaaaac caccctggcg cccaatacgc aaaccgcctc tccccgcgcg    1140
   ttggccgatt cattaatgca gctggcacga caggtttccc gactggaaag cgggcagtga 1200
   gcgcaacgca attaatgtaa gttagctcac tcattaggca ccccaggctt tacactttat 1260
   gcttccggct cgtataatgt gtggaattgt gagcggataa caatttcaca caggaaacag 1320
   ctatgaccat gattacggat tcactggccg tcgttttaca acgtcgtgac tgggaaaacc 1380
   ctggcgttac ccaacttaat cgccttgcag cacatccccc tttcgccagc tggcgtaata 1440
   gcgaagaggc ccgcaccgat cgcccttccc aacagttgcg cagcctgaat ggcgaatggc 1500
   gctttgcctg gtttccggca ccagaagcgg tgccggaaag ctggctggag tgcgatcttc 1560
   ctgaggccga tactgtcgtc gtcccctcaa actggcagat gcacggttac gatgcgccca 1620
   tctacaccaa cgtgacctat cccattacgg tcaatccgcc gtttgttccc acggagaatc 1680
   cgacgggttg ttactcgctc acatttaatg ttgatgaaag ctggctacag gaaggccaga 1740
   cgcgaattat ttttgatggc gtcgggatct gatccggatt tactaactgg aagaggcact 1800
   aaatgaacac gattaacatc gctaagaacg acttctctga catcgaactg gctgctatcc 1860
   cgttcaacac tctggctgac cattacggtg agcgtttagc tcgcgaacag ttggcccttg 1920
   agcatgagtc ttacgagatg ggtgaagcac gcttccgcaa gatgtttgag cgtcaactta 1980
   aagctggtga ggttgcggat aacgctgccg ccaagcctct catcactacc ctactcccta 2040
   agatgattgc acgcatcaac gactggtttg aggaagtgaa agctaagcgc ggcaagcgcc 2100
   cgacagcctt ccagttcctg caagaaatca agccggaagc cgtagcgtac atcaccatta 2160
   agaccactct ggcttgccta accagtgctg acaatacaac cgttcaggct gtagcaagcg 2220
   caatcggtcg ggccattgag gacgaggctc gcttcggtcg tatccgtgac cttgaagcta 2280
   agcacttcaa gaaaaacgtt gaggaacaac tcaacaagcg cgtagggcac gtctacaaga 2340
   aagcatttat gcaagttgtc gaggctgaca tgctctctaa gggtctactc ggtggcgagg 2400
   cgtggtcttc gtggcataag gaagactcta ttcatgtagg agtacgctgc atcgagatgc 2460
   tcattgagtc aaccggaatg gttagcttac accgccaaaa tgctggcgta gtaggtcaag 2520
   actctgagac tatcgaactc gcacctgaat acgctgaggc tatcgcaacc cgtgcaggtg 2580
   cgctggctgg catctctccg atgttccaac cttgcgtagt tcctcctaag ccgtggactg 2640
   gcattactgg tggtggctat tgggctaacg gtcgtcgtcc tctggcgctg gtgcgtactc 2700
   acagtaagaa agcactgatg cgctacgaag acgtttacat gcctgaggtg tacaaagcga 2760
   ttaacattgc gcaaaacacc gcatggaaaa tcaacaagaa agtcctagcg gtcgccaacg 2820
   taatcaccaa gtggaagcat tgtccggtcg aggacatccc tgcgattgag cgtgaagaac 2880
   tcccgatgaa accggaagac atcgacatga atcctgaggc tctcaccgcg tggaaacgtg 2940
   ctgccgctgc tgtgtaccgc aaggacaagg ctcgcaagtc tcgccgtatc agccttgagt      3000
   tcatgcttga gcaagccaat aagtttgcta accataaggc catctggttc ccttacaaca      3060
   tggactggcg cggtcgtgtt tacgctgtgt caatgttcaa cccgcaaggt aacgatatga      3120
   ccaaaggact gcttacgctg gcgaaaggta aaccaatcgg taaggaaggt tactactggc      3180
   tgaaaatcca cggtgcaaac tgtgcgggtg tcgataaggt tccgttccct gagcgcatca      3240
   agttcattga ggaaaaccac gagaacatca tggcttgcgc taagtctcca ctggagaaca      3300
   cttggtgggc tgagcaagat tctccgttct gcttccttgc gttctgcttt gagtacgctg      3360
   gggtacagca ccacggcctg agctataact gctcccttcc gctggcgttt gacgggtctt      3420
   gctctggcat ccagcacttc tccgcgatgc tccgagatga ggtaggtggt cgcgcggtta      3480
   acttgcttcc tagtgaaacc gttcaggaca tctacgggat tgttgctaag aaagtcaacg      3540
   agattctaca agcagacgca atcaatggga ccgataacga agtagttacc gtgaccgatg      3600
   agaacactgg tgaaatctct gagaaagtca agctgggcac taaggcactg gctggtcaat      3660
   ggctggctta cggtgttact cgcagtgtga ctaagcgttc agtcatgacg ctggcttacg      3720
   ggtccaaaga gttcggcttc cgtcaacaag tgctggaaga taccattcag ccagctattg      3780
   attccggcaa gggtctgatg ttcactcagc cgaatcaggc tgctggatac atggctaagc      3840
   tgatttggga atctgtgagc gtgacggtgg tagctgcggt tgaagcaatg aactggctta      3900
   agtctgctgc taagctgctg gctgctgagg tcaaagataa gaagactgga gagattcttc      3960
   gcaagcgttg cgctgtgcat tgggtaactc ctgatggttt ccctgtgtgg caggaataca      4020
   agaagcctat tcagacgcgc ttgaacctga tgttcctcgg tcagttccgc ttacagccta      4080
   ccattaacac caacaaagat agcgagattg atgcacacaa acaggagtct ggtatcgctc      4140
   ctaactttgt acacagccaa gacggtagcc accttcgtaa gactgtagtg tgggcacacg      4200
   agaagtacgg aatcgaatct tttgcactga ttcacgactc cttcggtacc attccggctg      4260
   acgctgcgaa cctgttcaaa gcagtgcgcg aaactatggt tgacacatat gagtcttgtg      4320
   atgtactggc tgatttctac gaccagttcg ctgaccagtt gcacgagtct caattggaca      4380
   aaatgccagc acttccggct aaaggtaact tgaacctccg tgacatctta gagtcggact      4440
   tcgcgttcgc gtaaaagctt gcgattgtgt aggctggagc tgcttcgaag ttcctatact      4500
   ttctagagaa taggaacttc ggaataggaa cttcaagatc ccctcacgct gccgcaagca      4560
   ctcagggcgc aagggctgct aaaggaagcg gaacacgtag aaagccagtc cgcagaaacg      4620
   gtgctgaccc cggatgaatg tcagctactg ggctatctgg acaagggaaa acgcaagcgc      4680
   aaagagaaag caggtagctt gcagtgggct tacatggcga tagctagact gggcggtttt      4740
   atggacagca agcgaaccgg aattgccagc tggggcgccc tctggtaagg ttgggaagcc 4800
   ctgcaaagta aactggatgg ctttcttgcc gccaaggatc tgatggcgca ggggatcaag 4860
   atctgatcaa gagacaggat gaggatcgtt tcgcatgatt gaacaagatg gattgcacgc 4920
   aggttctccg gccgcttggg tggagaggct attcggctat gactgggcac aacagacaat 4980
   cggctgctct gatgccgccg tgttccggct gtcagcgcag gggcgcccgg ttctttttgt 5040
   caagaccgac ctgtccggtg ccctgaatga actgcaggac gaggcagcgc ggctatcgtg 5100
   gctggccacg acgggcgttc cttgcgcagc tgtgctcgac gttgtcactg aagcgggaag 5160
   ggactggctg ctattgggcg aagtgccggg gcaggatctc ctgtcatctc accttgctcc 5220
   tgccgagaaa gtatccatca tggctgatgc aatgcggcgg ctgcatacgc ttgatccggc 5280
   tacctgccca ttcgaccacc aagcgaaaca tcgcatcgag cgagcacgta ctcggatgga 5340
   agccggtctt gtcgatcagg atgatctgga cgaagagcat caggggctcg cgccagccga 5400
   actgttcgcc aggctcaagg cgcgcatgcc cgacggcgag gatctcgtcg tgacccatgg 5460
   cgatgcctgc ttgccgaata tcatggtgga aaatggccgc ttttctggat tcatcgactg 5520
   tggccggctg ggtgtggcgg accgctatca ggacatagcg ttggctaccc gtgatattgc 5580
   tgaagagctt ggcggcgaat gggctgaccg cttcctcgtg ctttacggta tcgccgctcc 5640
   cgattcgcag cgcatcgcct tctatcgcct tcttgacgag ttcttctgag cgggactctg 5700
   gggttcgaaa tgaccgacca agcgacgccc aacctgccat cacgagattt cgattccacc 5760
   gccgccttct atgaaaggtt gggcttcgga atcgttttcc gggacgccgg ctggatgatc 5820
   ctccagcgcg gggatctcat gctggagttc ttcgcccacc ccagcttcaa aagcgctctg 5880
   aagttcctat actttctaga gaataggaac ttcggaatag gaactaagga ggatattcat 5940
   atggaccatg ggattcacaa ggccattgac gcatcgcccg gttagtttta 5990
<210> 2
   <211> 34
   <212> DNA
   <213> Artificial Sequence
<220>
   <223> Primer
<400> 2
   taaaaagctt gactcctgtt gatagatcca gtaa 34
<210> 3
   <211> 31
   <212> DNA
   <213> Artificial Sequence
<220>
   <223> Primer
<400> 3
   aaaacatgta ttctcaccaa taaaaaacgc c 31
<210> 4
   <211> 25
   <212> DNA
   <213> Artificial Sequence
<220>
   <223> Primer
<400> 4
   aagggcccgc cacgttgtgt gtctc 25
<210> 5
   <211> 31
   <212> DNA
   <213> Artificial Sequence
<220>
   <223> Primer
<400> 5
   aaagctagcg atatcgccgt cccgtcaagt c 31
<210> 6
   <211> 28
   <212> DNA
   <213> Artificial Sequence
<220>
   <223> Primer
<400> 6
   aaagctagcg ggaataaggg cgacacgg 28
<210> 7
   <211> 30
   <212> DNA
   <213> Artificial Sequence
<220>
   <223> Primer
<400> 7
   aaagggccca cgtgagtttt cgttccactg 30
<210> 8
   <211> 39
   <212> DNA
   <213> Artificial Sequence
<220>
   <223> Primer
<400> 8
   caatggtcag aaattggtta attggttgta acactggca 39
<210> 9
   <211> 23
   <212> DNA
   <213> Artificial Sequence
<220>
   <223> Primer
<400> 9
   gggcccacgt gagttttcgt tcc 23
<210> 10
   <211> 31
   <212> DNA
   <213> Artificial Sequence
<220>
   <223> Primer
<400> 10
   caattggcca cgttgtgtgt ctcaaaatct c 31
<210> 11
   <211> 28
   <212> DNA
   <213> Artificial Sequence
<220>
   <223> Primer
<400> 11
   gggcccgata tcgccgtccc gtcaagtc 28
<210> 12
   <211> 29
   <212> DNA
   <213> Artificial Sequence
<220>
   <223> Primer
<400> 12
   aaagggcccg caaccgacga cagtcctgc 29
<210> 13
   <211> 29
   <212> DNA
   <213> Artificial Sequence
<220>
   <223> Primer
<400> 13
   aaagggcccc ggtggacaaa ggttaaaac 29
<210> 14
   <211> 27
   <212> DNA
   <213> Artificial Sequence
<220>
   <223> Primer
<400> 14
   aaagctagcg acaccatcga atggcgc 27
<210> 15
   <211> 25
   <212> DNA
   <213> Artificial Sequence
<220>
   <223> Primer
<400> 15
   aaagctagct cactgcccgc tttcc 25
<210> 16
   <211> 33
   <212> DNA
   <213> Artificial Sequence
<220>
   <223> Primer
<400> 16
   aaacaattgg aaattaatac gactcactat agg 33
<210> 17
   <211> 40
   <212> DNA
   <213> Artificial Sequence
<220>
   <223> Primer
<400> 17
   aaagaattct ctccttctta aagttaaaca aaattatttc 40
<210> 18
   <211> 27
   <212> DNA
   <213> Artificial Sequence
<220>
   <223> Primer
<400> 18
   acggctacac tagaaggaca gtatttg 27
<210> 19
   <211> 20
   <212> DNA
   <213> Artificial Sequence
<220>
   <223> Primer
<400> 19
   agttaggcca ccacttcaag 20
<210> 20
   <211> 6357
   <212> DNA
   <213> Artificial Sequence
<220>
   <223> Expressionsvektor
<400> 20
   gaattggaaa ttaatacgac tcactatagg ggaattgtga gcggataaca attcccctct 60
   agaaataatt ttgtttaact ttaagaagga gagaattcga gctcggtacc cggggatcct 120
   ctagagtcga cctgcaggca tgcaagcttg actcctgttg atagatccag taatgacctc 180
   agaactccat ctggatttgt tcagaacgct cggttgccgc cgggcgtttt ttattggtga 240
   gaatacatgt gagcaaaagg ccagcaaaag gccaggaacc gtaaaaaggc cgcgttgctg 300
   gcgtttttcc ataggctccg cccccctgac gagcatcaca aaaatcgacg ctcaagtcag 360
   aggtggcgaa acccgacagg actataaaga taccaggcgt ttccccctgg aagctccctc 420
   gtgcgctctc ctgttccgac cctgccgctt accggatacc tgtccgcctt tctcccttcg 480
   ggaagcgtgg cgctttctca tagctcacgc tgtaggtatc tcagttcggt gtaggtcgtt 540
   cgctccaagc tgggctgtgt gcacgaaccc cccgttcagc ccgaccgctg cgccttatcc 600
   ggtaactatc gtcttgagtc caacccggta agacacgact tatcgccact ggcagcagcc 660
   actggtaaca ggattagcag agcgaggtat gtaggcggtg ctacagagtt cttgaagtgg 720
   tggcctaact acggctacac tagaaggaca gtatttggta tctgcgctct gctgaagcca 780
   gttaccttcg gaaaaagagt tggtagctct tgatccggca aacaaaccac cgctggtagc 840
   ggtggttttt ttgtttgcaa gcagcagatt acgcgcagaa aaaaaggatc tcaagaagat 900
   cctttgatct tttctacggg gtctgacgct cagtggaacg aaaactcacg tgggccgggc 960
   ccgcaaccga cgacagtcct gctaaaacgt tcgtttgata tcatttttcc taaaattgaa      1020
   tggcagagaa tcatgagtga cagccagacg ctggtggtaa aactcggcac cagtgtgcta      1080
   acaggcggat cgcgccgtct gaaccgtgcc catatcgttg aacttgttcg ccagtgcgcg      1140
   cagttacatg ccgccgggca tcggattgtt attgtgacgt cgggcgcgat cgccgccgga      1200
   cgtgagcacc tgggttaccc ggaactgcca gcgaccatcg cctcgaaaca actgctggcg      1260
   gcggtagggc agagtcgact gattcaactg tgggaacagc tgttttcgat ttatggcatt      1320
   cacgtcgggc aaatgctgct gacccgtgct gatatggaag accgtgaacg cttcctgaac      1380
   gcccgcgaca ccctgcgagc gttgctcgat aacaatatcg ttccggtaat caatgagaac      1440
   gatgctgtcg ctacggcaga gattaaggtc ggcgataacg ataacctttc tgcgctggcg      1500
   gcgattcttg cgggtgccga taaactgttg ctgctgaccg atcaaaaagg tttgtatacc      1560
   gctgacccgc gcagcaatcc gcaggcagaa ctgattaaag atgtttacgg cattgatgac      1620
   gcactgcgcg cgattgccgg tgacagcgtt tcaggcctcg gaactggcgg catgagtacc      1680
   aaattgcagg ccgctgacgt ggcttgccgt gcgggtatcg acaccattat tgccgcgggc      1740
   agcaagccgg gcgttattgg tgatgtgatg gaaggcattt ccgtcggtac gctgttccat      1800
   gcccaggcga ctccgcttga aaaccgtaaa cgctggattt tcggtgcgcc gccggcgggt      1860
   gaaatcacgg tagatgaagg ggcaactgcc gccattctgg aacgcggcag ctccctgttg      1920
   ccgaaaggca ttaaaagcgt gactggcaat ttctcgcgtg gtgaagtcat ccgcatttgc      1980
   aacctcgaag gccgcgatat cgcccacggc gtcagtcgtt acaacagcga tgcattacgc      2040
   cgtattgccg gacaccactc gcaagaaatt gatgcaatac tgggatatga atacggcccg      2100
   gttgccgttc accgtgatga catgattacc cgttaaggag caggctgatg ctggaacaaa      2160
   tgggcattgc cgcgaagcaa gcctcgtata aattagcgca actctccagc cgcgaaaaaa      2220
   atcgcgtgct ggaaaaaatc gccgatgaac tggaagcaca aagcgaaatc atcctcaacg      2280
   ctaacgccca ggatgttgct gacgcgcgag ccaatggcct tagcgaagcg atgcttgacc      2340
   gtctggcact gacgcccgca cggctgaaag gcattgccga cgatgtacgt caggtgtgca      2400
   acctcgccga tccggtgggg caggtaatcg atggcggcgt actggacagc ggcctgcgtc      2460
   ttgagcgtcg tcgcgtaccg ctgggggtta ttggcgtgat ttatgaagcg cgcccgaacg      2520
   tgacggttga tgtcgcttcg ctgtgcctga aaaccggtaa tgcggtgatc ctgcgcggtg      2580
   gcaaagaaac gtgtcgcact aacgctgcaa cggtggcggt gattcaggac gccctgaaat      2640
   cctgcggctt accggcgggt gccgtgcagg cgattgataa tcctgaccgt gcgctggtca      2700
   gtgaaatgct gcgtatggat aaatacatcg acatgctgat cccgcgtggt ggcgctggtt      2760
   tgcataaact gtgccgtgaa cagtcgacaa tcccggtgat cacaggtggt ataggcgtat 2820
   gccatattta cgttgatgaa agtgtagaga tcgctgaagc attaaaagtg atcgtcaacg 2880
   cgaaaactca gcgtccgagc acatgtaata cggttgaaac gttgctggtg aataaaaaca 2940
   tcgccgatag cttcctgccc gcattaagca aacaaatggc ggaaagcggc gtgacattac 3000
   acgcagatgc agctgcactg gcgcagttgc aggcaggccc tgcgaaggtg gttgctgtta 3060
   aagccgaaga gtatgacgat gagtttctgt cattagattt gaacgtcaaa atcgtcagcg 3120
   atcttgacga tgccatcgcc catattcgtg aacacggcac acaacactcc gatgcgatcc 3180
   tgacccgcga tatgcgcaac gcccagcgtt ttgttaacga agtggattcg tccgctgttt 3240
   acgttaacgc ctctacgcgt tttaccgacg gcggccagtt tggtctgggt gcggaagtgg 3300
   cggtaagcac acaaaaactc cacgcgcgtg gcccaatggg gctggaagca ctgaccactt 3360
   acaagtggat cggcattggt gattacacca ttcgtgcgta aataaaaccg ggtgatgcaa 3420
   aagtagccat ttgattcaca aggccattga cgcatcgccc ggttagtttt aaccttgtcc 3480
   accggggccc gatatcgccg tcccgtcaag tcagcgtaat gctctgccag tgttacaacc 3540
   aattaaccaa ttctgattag aaaaactcat cgagcatcaa atgaaactgc aatttattca 3600
   tatcaggatt atcaatacca tatttttgaa aaagccgttt ctgtaatgaa ggagaaaact 3660
   caccgaggca gttccatagg atggcaagat cctggtatcg gtctgcgatt ccgactcgtc 3720
   caacatcaat acaacctatt aatttcccct cgtcaaaaat aaggttatca agtgagaaat 3780
   caccatgagt gacgactgaa tccggtgaga atggcaaaag cttatgcatt tctttccaga 3840
   cttgttcaac aggccagcca ttacgctcgt catcaaaatc actcgcatca accaaaccgt 3900
   tattcattcg tgattgcgcc tgagcgagac gaaatacgcg atcgctgtta aaaggacaat 3960
   tacaaacagg aatcgaatgc aaccggcgca ggaacactgc cagcgcatca acaatatttt 4020
   cacctgaatc aggatattct tctaatacct ggaatgctgt tttcccgggg atcgcagtgg 4080
   tgagtaacca tgcatcatca ggagtacgga taaaatgctt gatggtcgga agaggcataa 4140
   attccgtcag ccagtttagt ctgaccatct catctgtaac atcattggca acgctacctt 4200
   tgccatgttt cagaaacaac tctggcgcat cgggcttccc atacaatcga tagattgtcg 4260
   cacctgattg cccgacatta tcgcgagccc atttataccc atataaatca gcatccatgt 4320
   tggaatttaa tcgcggcctc gagcaagacg tttcccgttg aatatggctc ataacacccc 4380
   ttgtattact gtttatgtaa gcagacagtt ttattgttca tgatgatata tttttatctt 4440
   gtgcaatgta acatcagaga ttttgagaca cacaacgtgg ccaattgtca gaattggtta 4500
   attggttgta acactggcag agcattacgc tgacttgacg ggacggcgat atcgctagcg 4560
   acaccatcga atggcgcaaa acctttcgcg gtatggcatg atagcgcccg gaagagagtc 4620
   aattcagggt ggtgaatgtg aaaccagtaa cgttatacga tgtcgcagag tatgccggtg 4680
   tctcttatca gaccgtttcc cgcgtggtga accaggccag ccacgtttct gcgaaaacgc 4740
   gggaaaaagt ggaagcggcg atggcggagc tgaattacat tcccaaccgc gtggcacaac 4800
   aactggcggg caaacagtcg ttgctgattg gcgttgccac ctccagtctg gccctgcacg 4860
   cgccgtcgca aattgtcgcg gcgattaaat ctcgcgccga tcaactgggt gccagcgtgg 4920
   tggtgtcgat ggtagaacga agcggcgtcg aagcctgtaa agcggcggtg cacaatcttc 4980
   tcgcgcaacg cgtcagtggg ctgatcatta actatccgct ggatgaccag gatgccattg 5040
   ctgtggaagc tgcctgcact aatgttccgg cgttatttct tgatgtctct gaccagacac 5100
   ccatcaacag tattattttc tcccatgaag acggtacgcg actgggcgtg gagcatctgg 5160
   tcgcattggg tcaccagcaa atcgcgctgt tagcgggccc attaagttct gtctcggcgc 5220
   gtctgcgtct ggctggctgg cataaatatc tcactcgcaa tcaaattcag ccgatagcgg 5280
   aacgggaagg cgactggagt gccatgtccg gttttcaaca aaccatgcaa atgctgaatg 5340
   agggcatcgt tcccactgcg atgctggttg ccaacgatca gatggcgctg ggcgcaatgc 5400
   gcgccattac cgagtccggg ctgcgcgttg gtgcggatat ctcggtagtg ggatacgacg 5460
   ataccgaaga cagctcatgt tatatcccgc cgttaaccac catcaaacag gattttcgcc 5520
   tgctggggca aaccagcgtg gaccgcttgc tgcaactctc tcagggccag gcggtgaagg 5580
   gcaatcagct gttgcccgtc tcactggtga aaagaaaaac caccctggcg cccaatacgc 5640
   aaaccgcctc tccccgcgcg ttggccgatt cattaatgca gctggcacga caggtttccc 5700
   gactggaaag cgggcagtga gctagcggga ataagggcga cacggaaatg ttgaatactc 5760
   atactcttcc tttttcaata ttattgaagc atttatcagg gttattgtct catgagcgga 5820
   tacatatttg aatgtattta gaaaaataaa caaatagggg ttccgcgcac atttccccga 5880
   aaagtgccac ctgacgtcta agaaaccatt attatcatga cattaaccta taaaaatagg 5940
   cgtatcacga ggccctttcg tctcgcgcgt ttcggtgatg acggtgaaaa cctctgacac 6000
   atgcagctcc cggagacggt cacagcttgt ctgtaagcgg atgccgggag cagacaagcc 6060
   cgtcagggcg cgtcagcggg tgttggcggg tgtcggggct ggcttaacta tgcggcatca 6120
   gagcagattg tactgagagt gcaccatatg cggtgtgaaa taccgcacag atgcgtaagg 6180
   agaaaatacc gcatcaggcg ccattcgcca ttcaggctgc gcaactgttg ggaagggcga 6240
   tcggtgcggg cctcttcgct attacgccag ctggcgaaag ggggatgtgc tgcaaggcga 6300
   ttaagttggg taacgccagg gttttcccag tcacgacgtt gtaaaacgac ggccagt 6357
<210> 21
   <211> 32
   <212> DNA
   <213> Artificial Sequence
<220>
   <223> Primer
<400> 21
   aaacatatgg tgaaaccatg aaaaatggca gc 32
<210> 22
   <211> 28
   <212> DNA
   <213> Artificial Sequence
<220>
   <223> Primer
<400> 22
   aaacatatgc tcgatggcta cgagggca 28
<210> 23
   <211> 6604
   <212> DNA
   <213> Artificial Sequence
<220>
   <223> Expressionsvektor
<400> 23
   gaattggaaa ttaatacgac tcactatagg ggaattgtga gcggataaca attcccctct        60
   agaaataatt ttgtttaact ttaagaagga gagaattcga gctcggtacc cggggatcct       120
   ctagagtcga cctgcaggca tgcaagcttg actcctgttg atagatccag taatgacctc       180
   agaactccat ctggatttgt tcagaacgct cggttgccgc cgggcgtttt ttattggtga       240
   gaatacatgt gagcaaaagg ccagcaaaag gccaggaacc gtaaaaaggc cgcgttgctg       300
   gcgtttttcc ataggctccg cccccctgac gagcatcaca aaaatcgacg ctcaagtcag       360
   aggtggcgaa acccgacagg actataaaga taccaggcgt ttccccctgg aagctccctc       420
   gtgcgctctc ctgttccgac cctgccgctt accggatacc tgtccgcctt tctcccttcg       480
   ggaagcgtgg cgctttctca tagctcacgc tgtaggtatc tcagttcggt gtaggtcgtt       540
   cgctccaagc tgggctgtgt gcacgaaccc cccgttcagc ccgaccgctg cgccttatcc       600
   ggtaactatc gtcttgagtc caacccggta agacacgact tatcgccact ggcagcagcc       660
   actggtaaca ggattagcag agcgaggtat gtaggcggtg ctacagagtt cttgaagtgg       720
   tggcctaact acggctacac tagaaggaca gtatttggta tctgcgctct gctgaagcca       780
   gttaccttcg gaaaaagagt tggtagctct tgatccggca aacaaaccac cgctggtagc 840
   ggtggttttt ttgtttgcaa gcagcagatt acgcgcagaa aaaaaggatc tcaagaagat 900
   cctttgatct tttctacggg gtctgacgct cagtggaacg aaaactcacg tgggccgggc 960
   ccgcaaccga cgacagtcct gctaaaacgt tcgtttgata tcatttttcc taaaattgaa 1020
   tggcagagaa tcatgagtga cagccagacg ctggtggtaa aactcggcac cagtgtgcta 1080
   acaggcggat cgcgccgtct gaaccgtgcc catatcgttg aacttgttcg ccagtgcgcg 1140
   cagttacatg ccgccgggca tcggattgtt attgtgacgt cgggcgcgat cgccgccgga 1200
   cgtgagcacc tgggttaccc ggaactgcca gcgaccatcg cctcgaaaca actgctggcg 1260
   gcggtagggc agagtcgact gattcaactg tgggaacagc tgttttcgat ttatggcatt 1320
   cacgtcgggc aaatgctgct gacccgtgct gatatggaag accgtgaacg cttcctgaac 1380
   gcccgcgaca ccctgcgagc gttgctcgat aacaatatcg ttccggtaat caatgagaac 1440
   gatgctgtcg ctacggcaga gattaaggtc ggcgataacg ataacctttc tgcgctggcg 1500
   gcgattcttg cgggtgccga taaactgttg ctgctgaccg atcaaaaagg tttgtatacc 1560
   gctgacccgc gcagcaatcc gcaggcagaa ctgattaaag atgtttacgg cattgatgac 1620
   gcactgcgcg cgattgccgg tgacagcgtt tcaggcctcg gaactggcgg catgagtacc 1680
   aaattgcagg ccgctgacgt ggcttgccgt gcgggtatcg acaccattat tgccgcgggc 1740
   agcaagccgg gcgttattgg tgatgtgatg gaaggcattt ccgtcggtac gctgttccat 1800
   gcccaggcga ctccgcttga aaaccgtaaa cgctggattt tcggtgcgcc gccggcgggt 1860
   gaaatcacgg tagatgaagg ggcaactgcc gccattctgg aacgcggcag ctccctgttg 1920
   ccgaaaggca ttaaaagcgt gactggcaat ttctcgcgtg gtgaagtcat ccgcatttgc 1980
   aacctcgaag gccgcgatat cgcccacggc gtcagtcgtt acaacagcga tgcattacgc 2040
   cgtattgccg gacaccactc gcaagaaatt gatgcaatac tgggatatga atacggcccg 2100
   gttgccgttc accgtgatga catgattacc cgttaaggag caggctgatg ctggaacaaa 2160
   tgggcattgc cgcgaagcaa gcctcgtata aattagcgca actctccagc cgcgaaaaaa 2220
   atcgcgtgct ggaaaaaatc gccgatgaac tggaagcaca aagcgaaatc atcctcaacg 2280
   ctaacgccca ggatgttgct gacgcgcgag ccaatggcct tagcgaagcg atgcttgacc 2340
   gtctggcact gacgcccgca cggctgaaag gcattgccga cgatgtacgt caggtgtgca 2400
   acctcgccga tccggtgggg caggtaatcg atggcggcgt actggacagc ggcctgcgtc 2460
   ttgagcgtcg tcgcgtaccg ctgggggtta ttggcgtgat ttatgaagcg cgcccgaacg 2520
   tgacggttga tgtcgcttcg ctgtgcctga aaaccggtaa tgcggtgatc ctgcgcggtg 2580
   gcaaagaaac gtgtcgcact aacgctgcaa cggtggcggt gattcaggac gccctgaaat   2640
   cctgcggctt accggcgggt gccgtgcagg cgattgataa tcctgaccgt gcgctggtca   2700
   gtgaaatgct gcgtatggat aaatacatcg acatgctgat cccgcgtggt ggcgctggtt   2760
   tgcataaact gtgccgtgaa cagtcgacaa tcccggtgat cacaggtggt ataggcgtat   2820
   gccatattta cgttgatgaa agtgtagaga tcgctgaagc attaaaagtg atcgtcaacg   2880
   cgaaaactca gcgtccgagc acatgtaata cggttgaaac gttgctggtg aataaaaaca   2940
   tcgccgatag cttcctgccc gcattaagca aacaaatggc ggaaagcggc gtgacattac   3000
   acgcagatgc agctgcactg gcgcagttgc aggcaggccc tgcgaaggtg gttgctgtta   3060
   aagccgaaga gtatgacgat gagtttctgt cattagattt gaacgtcaaa atcgtcagcg   3120
   atcttgacga tgccatcgcc catattcgtg aacacggcac acaacactcc gatgcgatcc   3180
   tgacccgcga tatgcgcaac gcccagcgtt ttgttaacga agtggattcg tccgctgttt   3240
   acgttaacgc ctctacgcgt tttaccgacg gcggccagtt tggtctgggt gcggaagtgg   3300
   cggtaagcac acaaaaactc cacgcgcgtg gcccaatggg gctggaagca ctgaccactt   3360
   acaagtggat cggcattggt gattacacca ttcgtgcgta aataaaaccg ggtgatgcaa   3420
   aagtagccat ttgattcaca aggccattga cgcatcgccc ggttagtttt aaccttgtcc   3480
   accggggccc gatatcgccg tcccgtcaag tcagcgtaat gctctgccag tgttacaacc   3540
   aattaaccaa ttctgattag aaaaactcat cgagcatcaa atgaaactgc aatttattca   3600
   tatcaggatt atcaatacca tatttttgaa aaagccgttt ctgtaatgaa ggagaaaact   3660
   caccgaggca gttccatagg atggcaagat cctggtatcg gtctgcgatt ccgactcgtc   3720
   caacatcaat acaacctatt aatttcccct cgtcaaaaat aaggttatca agtgagaaat   3780
   caccatgagt gacgactgaa tccggtgaga atggcaaaag cttatgcatt tctttccaga   3840
   cttgttcaac aggccagcca ttacgctcgt catcaaaatc actcgcatca accaaaccgt   3900
   tattcattcg tgattgcgcc tgagcgagac gaaatacgcg atcgctgtta aaaggacaat   3960
   tacaaacagg aatcgaatgc aaccggcgca ggaacactgc cagcgcatca acaatatttt   4020
   cacctgaatc aggatattct tctaatacct ggaatgctgt tttcccgggg atcgcagtgg   4080
   tgagtaacca tgcatcatca ggagtacgga taaaatgctt gatggtcgga agaggcataa   4140
   attccgtcag ccagtttagt ctgaccatct catctgtaac atcattggca acgctacctt   4200
   tgccatgttt cagaaacaac tctggcgcat cgggcttccc atacaatcga tagattgtcg   4260
   cacctgattg cccgacatta tcgcgagccc atttataccc atataaatca gcatccatgt   4320
   tggaatttaa tcgcggcctc gagcaagacg tttcccgttg aatatggctc ataacacccc   4380
   ttgtattact gtttatgtaa gcagacagtt ttattgttca tgatgatata tttttatctt 4440
   gtgcaatgta acatcagaga ttttgagaca cacaacgtgg ccaattgtca gaattggtta 4500
   attggttgta acactggcag agcattacgc tgacttgacg ggacggcgat atcgctagcg 4560
   acaccatcga atggcgcaaa acctttcgcg gtatggcatg atagcgcccg gaagagagtc 4620
   aattcagggt ggtgaatgtg aaaccagtaa cgttatacga tgtcgcagag tatgccggtg 4680
   tctcttatca gaccgtttcc cgcgtggtga accaggccag ccacgtttct gcgaaaacgc 4740
   gggaaaaagt ggaagcggcg atggcggagc tgaattacat tcccaaccgc gtggcacaac 4800
   aactggcggg caaacagtcg ttgctgattg gcgttgccac ctccagtctg gccctgcacg 4860
   cgccgtcgca aattgtcgcg gcgattaaat ctcgcgccga tcaactgggt gccagcgtgg 4920
   tggtgtcgat ggtagaacga agcggcgtcg aagcctgtaa agcggcggtg cacaatcttc 4980
   tcgcgcaacg cgtcagtggg ctgatcatta actatccgct ggatgaccag gatgccattg 5040
   ctgtggaagc tgcctgcact aatgttccgg cgttatttct tgatgtctct gaccagacac 5100
   ccatcaacag tattattttc tcccatgaag acggtacgcg actgggcgtg gagcatctgg 5160
   tcgcattggg tcaccagcaa atcgcgctgt tagcgggccc attaagttct gtctcggcgc 5220
   gtctgcgtct ggctggctgg cataaatatc tcactcgcaa tcaaattcag ccgatagcgg 5280
   aacgggaagg cgactggagt gccatgtccg gttttcaaca aaccatgcaa atgctgaatg 5340
   agggcatcgt tcccactgcg atgctggttg ccaacgatca gatggcgctg ggcgcaatgc 5400
   gcgccattac cgagtccggg ctgcgcgttg gtgcggatat ctcggtagtg ggatacgacg 5460
   ataccgaaga cagctcatgt tatatcccgc cgttaaccac catcaaacag gattttcgcc 5520
   tgctggggca aaccagcgtg gaccgcttgc tgcaactctc tcagggccag gcggtgaagg 5580
   gcaatcagct gttgcccgtc tcactggtga aaagaaaaac caccctggcg cccaatacgc 5640
   aaaccgcctc tccccgcgcg ttggccgatt cattaatgca gctggcacga caggtttccc 5700
   gactggaaag cgggcagtga gctagcggga ataagggcga cacggaaatg ttgaatactc 5760
   atactcttcc tttttcaata ttattgaagc atttatcagg gttattgtct catgagcgga 5820
   tacatatttg aatgtattta gaaaaataaa caaatagggg ttccgcgcac atttccccga 5880
   aaagtgccac ctgacgtcta agaaaccatt attatcatga cattaaccta taaaaatagg 5940
   cgtatcacga ggccctttcg tctcgcgcgt ttcggtgatg acggtgaaaa cctctgacac 6000
   atgcagctcc cggagacggt cacagcttgt ctgtaagcgg atgccgggag cagacaagcc 6060
   cgtcagggcg cgtcagcggg tgttggcggg tgtcggggct ggcttaacta tgcggcatca 6120
   gagcagattg tactgagagt gcaccatatg ctcgatggct acgagggcag acagtaagtg 6180
   gatttaccat aatcccttaa ttgtacgcac cgctaaaacg cgttcagcgc gatcacggca 6240
   gcagacaggt aaaaatggca acaaaccacc cgaaaaactg ccgcgatcgc gcctgataaa 6300
   ttttaaccgt atgaatacct atgcaaccag agggtacagg ccacattacc cccacttaat 6360
   ccactgaagc tgccattttt catggtttca ccatatgcgg tgtgaaatac cgcacagatg 6420
   cgtaaggaga aaataccgca tcaggcgcca ttcgccattc aggctgcgca actgttggga 6480
   agggcgatcg gtgcgggcct cttcgctatt acgccagctg gcgaaagggg gatgtgctgc 6540
   aaggcgatta agttgggtaa cgccagggtt ttcccagtca cgacgttgta aaacgacggc 6600
   cagt 6604
<210> 24
   <211> 30
   <212> DNA
   <213> Artificial Sequence
<220>
   <223> Primer
<400> 24
   aaaccatggg atatcgctag cgacaccatc 30
<210> 25
   <211> 30
   <212> DNA
   <213> Artificial Sequence
<220>
   <223> Primer
<400> 25
   actagtcggt ggacaaggtt aaaactaacc 30
<210> 26
   <211> 34
   <212> DNA
   <213> Artificial Sequence
<220>
   <223> Primer
<400> 26
   aaaccatggt ctcatgtttg acagcttatc atcg 34
<210> 27
   <211> 29
   <212> DNA
   <213> Artificial Sequence
<220>
   <223> Primer
<400> 27
   aaaactagtc tgtcagacca agtttactc 29
<210> 28
   <211> 27
   <212> DNA
   <213> Artificial Sequence
<220>
   <223> Primer
<400> 28
   ccatgggata tcgctagcga caccatc 27
<210> 29
   <211> 25
   <212> DNA
   <213> Artificial Sequence
<220>
   <223> Primer
<400> 29
   tctcatgttt gacagcttat catcg 25
<210> 30
   <211> 6920
   <212> DNA
   <213> Artificial Sequence
<220>
   <223> Expressionsvektor
<400> 30
   gaattggaaa ttaatacgac tcactatagg ggaattgtga gcggataaca attcccctct 60
   agaaataatt ttgtttaact ttaagaagga gagaattcga gctcggtacc cggggatcct 120
   ctagagtcga cctgcaggca tgcaagcttg actcctgttg atagatccag taatgacctc 180
   agaactccat ctggatttgt tcagaacgct cggttgccgc cgggcgtttt ttattggtga 240
   gaatacatgt gagcaaaagg ccagcaaaag gccaggaacc gtaaaaaggc cgcgttgctg 300
   gcgtttttcc ataggctccg cccccctgac gagcatcaca aaaatcgacg ctcaagtcag 360
   aggtggcgaa acccgacagg actataaaga taccaggcgt ttccccctgg aagctccctc 420
   gtgcgctctc ctgttccgac cctgccgctt accggatacc tgtccgcctt tctcccttcg 480
   ggaagcgtgg cgctttctca tagctcacgc tgtaggtatc tcagttcggt gtaggtcgtt 540
   cgctccaagc tgggctgtgt gcacgaaccc cccgttcagc ccgaccgctg cgccttatcc 600
   ggtaactatc gtcttgagtc caacccggta agacacgact tatcgccact ggcagcagcc 660
   actggtaaca ggattagcag agcgaggtat gtaggcggtg ctacagagtt cttgaagtgg     720
   tggcctaact acggctacac tagaaggaca gtatttggta tctgcgctct gctgaagcca     780
   gttaccttcg gaaaaagagt tggtagctct tgatccggca aacaaaccac cgctggtagc     840
   ggtggttttt ttgtttgcaa gcagcagatt acgcgcagaa aaaaaggatc tcaagaagat     900
   cctttgatct tttctacggg gtctgacgct cagtggaacg aaaactcacg tgggccgggc     960
   ccgcaaccga cgacagtcct gctaaaacgt tcgtttgata tcatttttcc taaaattgaa    1020
   tggcagagaa tcatgagtga cagccagacg ctggtggtaa aactcggcac cagtgtgcta    1080
   acaggcggat cgcgccgtct gaaccgtgcc catatcgttg aacttgttcg ccagtgcgcg    1140
   cagttacatg ccgccgggca tcggattgtt attgtgacgt cgggcgcgat cgccgccgga    1200
   cgtgagcacc tgggttaccc ggaactgcca gcgaccatcg cctcgaaaca actgctggcg    1260
   gcggtagggc agagtcgact gattcaactg tgggaacagc tgttttcgat ttatggcatt    1320
   cacgtcgggc aaatgctgct gacccgtgct gatatggaag accgtgaacg cttcctgaac    1380
   gcccgcgaca ccctgcgagc gttgctcgat aacaatatcg ttccggtaat caatgagaac    1440
   gatgctgtcg ctacggcaga gattaaggtc ggcgataacg ataacctttc tgcgctggcg    1500
   gcgattcttg cgggtgccga taaactgttg ctgctgaccg atcaaaaagg tttgtatacc    1560
   gctgacccgc gcagcaatcc gcaggcagaa ctgattaaag atgtttacgg cattgatgac    1620
   gcactgcgcg cgattgccgg tgacagcgtt tcaggcctcg gaactggcgg catgagtacc    1680
   aaattgcagg ccgctgacgt ggcttgccgt gcgggtatcg acaccattat tgccgcgggc    1740
   agcaagccgg gcgttattgg tgatgtgatg gaaggcattt ccgtcggtac gctgttccat    1800
   gcccaggcga ctccgcttga aaaccgtaaa cgctggattt tcggtgcgcc gccggcgggt    1860
   gaaatcacgg tagatgaagg ggcaactgcc gccattctgg aacgcggcag ctccctgttg    1920
   ccgaaaggca ttaaaagcgt gactggcaat ttctcgcgtg gtgaagtcat ccgcatttgc    1980
   aacctcgaag gccgcgatat cgcccacggc gtcagtcgtt acaacagcga tgcattacgc    2040
   cgtattgccg gacaccactc gcaagaaatt gatgcaatac tgggatatga atacggcccg    2100
   gttgccgttc accgtgatga catgattacc cgttaaggag caggctgatg ctggaacaaa    2160
   tgggcattgc cgcgaagcaa gcctcgtata aattagcgca actctccagc cgcgaaaaaa    2220
   atcgcgtgct ggaaaaaatc gccgatgaac tggaagcaca aagcgaaatc atcctcaacg    2280
   ctaacgccca ggatgttgct gacgcgcgag ccaatggcct tagcgaagcg atgcttgacc    2340
   gtctggcact gacgcccgca cggctgaaag gcattgccga cgatgtacgt caggtgtgca    2400
   acctcgccga tccggtgggg caggtaatcg atggcggcgt actggacagc ggcctgcgtc    2460
   ttgagcgtcg tcgcgtaccg ctgggggtta ttggcgtgat ttatgaagcg cgcccgaacg      2520
   tgacggttga tgtcgcttcg ctgtgcctga aaaccggtaa tgcggtgatc ctgcgcggtg      2580
   gcaaagaaac gtgtcgcact aacgctgcaa cggtggcggt gattcaggac gccctgaaat      2640
   cctgcggctt accggcgggt gccgtgcagg cgattgataa tcctgaccgt gcgctggtca      2700
   gtgaaatgct gcgtatggat aaatacatcg acatgctgat cccgcgtggt ggcgctggtt      2760
   tgcataaact gtgccgtgaa cagtcgacaa tcccggtgat cacaggtggt ataggcgtat      2820
   gccatattta cgttgatgaa agtgtagaga tcgctgaagc attaaaagtg atcgtcaacg      2880
   cgaaaactca gcgtccgagc acatgtaata cggttgaaac gttgctggtg aataaaaaca      2940
   tcgccgatag cttcctgccc gcattaagca aacaaatggc ggaaagcggc gtgacattac      3000
   acgcagatgc agctgcactg gcgcagttgc aggcaggccc tgcgaaggtg gttgctgtta      3060
   aagccgaaga gtatgacgat gagtttctgt cattagattt gaacgtcaaa atcgtcagcg      3120
   atcttgacga tgccatcgcc catattcgtg aacacggcac acaacactcc gatgcgatcc      3180
   tgacccgcga tatgcgcaac gcccagcgtt ttgttaacga agtggattcg tccgctgttt      3240
   acgttaacgc ctctacgcgt tttaccgacg gcggccagtt tggtctgggt gcggaagtgg      3300
   cggtaagcac acaaaaactc cacgcgcgtg gcccaatggg gctggaagca ctgaccactt      3360
   acaagtggat cggcattggt gattacacca ttcgtgcgta aataaaaccg ggtgatgcaa      3420
   aagtagccat ttgattcaca aggccattga cgcatcgccc ggttagtttt aaccttgtcc      3480
   accgactagt gttggtttgc gcattcacag ttctccgcaa gaattgattg gctccaattc      3540
   ttggagtggt gaatccgtta gcgaggtgcc gccggcttcc attcaggtcg aggtggcccg      3600
   gctccatgca ccgcgacgca acgcggggag gcagacaagg tatagggcgg cgcctacaat      3660
   ccatgccaac ccgttccatg tgctcgccga ggcggcataa atcgccgtga cgatcagcgg      3720
   tccaatgatc gaagttaggc tggtaagagc cgcgagcgat ccttgaagct gtccctgatg      3780
   gtcgtcatct acctgcctgg acagcatggc ctgcaacgcg ggcatcccga tgccgccgga      3840
   agcgagaaga atcataatgg ggaaggccat ccagcctcgc gtcgcgaacg ccagcaagac      3900
   gtagcccagc gcgtcggccg ccatgccggc gataatggcc tgcttctcgc cgaaacgttt      3960
   ggtggcggga ccagtgacga aggcttgagc gagggcgtgc aagattccga ataccgcaag      4020
   cgacaggccg atcatcgtcg cgctccagcg aaagcggtcc tcgccgaaaa tgacccagag      4080
   cgctgccggc acctgtccta cgagttgcat gataaagaag acagtcataa gtgcggcgac      4140
   gatagtcatg ccccgcgccc accggaagga gctgactggg ttgaaggctc tcaagggcat      4200
   cggtcgacgc tctcccttat gcgactcctg cattaggaag cagcccagta gtaggttgag      4260
   gccgttgagc accgccgccg caaggaatgg tgcatgcaag gagatggcgc ccaacagtcc 4320
   cccggccacg gggcctgcca ccatacccac gccgaaacaa gcgctcatga gcccgaagtg 4380
   gcgagcccga tcttccccat cggtgatgtc ggcgatatag gcgccagcaa ccgcacctgt 4440
   ggcgccggtg atgccggcca cgatgcgtcc ggcgtagagg atccacagga cgggtgtggt 4500
   cgccatgatc gcgtagtcga tagtggctcc aagtagcgaa gcgagcagga ctgggcggcg 4560
   gccaaagcgg tcggacagtg ctccgagaac gggtgcgcat agaaattgca tcaacgcata 4620
   tagcgctagc agcacgccat agtgactggc gatgctgtcg gaatggacga tatcccgcaa 4680
   gaggcccggc agtaccggca taaccaagcc tatgcctaca gcatccaggg tgacggtgcc 4740
   gaggatgacg atgagcgcat tgttagattt catacacggt gcctgactgc gttagcaatt 4800
   taactgtgat aaactaccgc attaaagctt atcgatgata agctgtcaaa catgagacca 4860
   tgggatatcg ctagcgacac catcgaatgg cgcaaaacct ttcgcggtat ggcatgatag 4920
   cgcccggaag agagtcaatt cagggtggtg aatgtgaaac cagtaacgtt atacgatgtc 4980
   gcagagtatg ccggtgtctc ttatcagacc gtttcccgcg tggtgaacca ggccagccac 5040
   gtttctgcga aaacgcggga aaaagtggaa gcggcgatgg cggagctgaa ttacattccc 5100
   aaccgcgtgg cacaacaact ggcgggcaaa cagtcgttgc tgattggcgt tgccacctcc 5160
   agtctggccc tgcacgcgcc gtcgcaaatt gtcgcggcga ttaaatctcg cgccgatcaa 5220
   ctgggtgcca gcgtggtggt gtcgatggta gaacgaagcg gcgtcgaagc ctgtaaagcg 5280
   gcggtgcaca atcttctcgc gcaacgcgtc agtgggctga tcattaacta tccgctggat 5340
   gaccaggatg ccattgctgt ggaagctgcc tgcactaatg ttccggcgtt atttcttgat 5400
   gtctctgacc agacacccat caacagtatt attttctccc atgaagacgg tacgcgactg 5460
   ggcgtggagc atctggtcgc attgggtcac cagcaaatcg cgctgttagc gggcccatta 5520
   agttctgtct cggcgcgtct gcgtctggct ggctggcata aatatctcac tcgcaatcaa 5580
   attcagccga tagcggaacg ggaaggcgac tggagtgcca tgtccggttt tcaacaaacc 5640
   atgcaaatgc tgaatgaggg catcgttccc actgcgatgc tggttgccaa cgatcagatg 5700
   gcgctgggcg caatgcgcgc cattaccgag tccgggctgc gcgttggtgc ggatatctcg 5760
   gtagtgggat acgacgatac cgaagacagc tcatgttata tcccgccgtt aaccaccatc 5820
   aaacaggatt ttcgcctgct ggggcaaacc agcgtggacc gcttgctgca actctctcag 5880
   ggccaggcgg tgaagggcaa tcagctgttg cccgtctcac tggtgaaaag aaaaaccacc 5940
   ctggcgccca atacgcaaac cgcctctccc cgcgcgttgg ccgattcatt aatgcagctg 6000
   gcacgacagg tttcccgact ggaaagcggg cagtgagcta gcgggaataa gggcgacacg 6060
   gaaatgttga atactcatac tcttcctttt tcaatattat tgaagcattt atcagggtta 6120
   ttgtctcatg agcggataca tatttgaatg tatttagaaa aataaacaaa taggggttcc 6180
   gcgcacattt ccccgaaaag tgccacctga cgtctaagaa accattatta tcatgacatt 6240
   aacctataaa aataggcgta tcacgaggcc ctttcgtctc gcgcgtttcg gtgatgacgg 6300
   tgaaaacctc tgacacatgc agctcccgga gacggtcaca gcttgtctgt aagcggatgc 6360
   cgggagcaga caagcccgtc agggcgcgtc agcgggtgtt ggcgggtgtc ggggctggct 6420
   taactatgcg gcatcagagc agattgtact gagagtgcac catatgctcg atggctacga 6480
   gggcagacag taagtggatt taccataatc ccttaattgt acgcaccgct aaaacgcgtt 6540
   cagcgcgatc acggcagcag acaggtaaaa atggcaacaa accacccgaa aaactgcgcc 6600
   gatcgcgcct gataaatttt aaccgtatga atacctatgc aaccagaggg tacaggccac 6660
   attaccccca cttaatccac tgaagctgcc atttttcatg gtttcaccat atgcggtgtg 6720
   aaataccgca cagatgcgta aggagaaaat accgcatcag gcgccattcg ccattcaggc 6780
   tgcgcaactg ttgggaaggg cgatcggtgc gggcctcttc gctattacgc cagctggcga 6840
   aagggggatg tgctgcaagg cgattaagtt gggtaacgcc agggttttcc cagtcacgac 6900
   gttgtaaaac gacggccagt 6920
<210> 31
   <211> 25
   <212> DNA
   <213> Artificial Sequence
<220>
   <223> Primer
<400> 31
   acgtgagttt tcgttccact gagcg 25
<210> 32
   <211> 20
   <212> DNA
   <213> Artificial Sequence
<220>
   <223> Primer
<400> 32
   gggcccgata tcgccgtccc 20
<210> 33
   <211> 4071
   <212> DNA
   <213> Artificial Sequence
<220>
   <223> Expressionsvektor
<400> 33
   gaattggaaa ttaatacgac tcactatagg ggaattgtga gcggataaca attcccctct      60
   agaaataatt ttgtttaact ttaagaagga gagaattcga gctcggtacc cggggatcct     120
   ctagagtcga cctgcaggca tgcaagcttg actcctgttg atagatccag taatgacctc     180
   agaactccat ctggatttgt tcagaacgct cggttgccgc cgggcgtttt ttattggtga     240
   gaatacatgt gagcaaaagg ccagcaaaag gccaggaacc gtaaaaaggc cgcgttgctg     300
   gcgtttttcc ataggctccg cccccctgac gagcatcaca aaaatcgacg ctcaagtcag     360
   aggtggcgaa acccgacagg actataaaga taccaggcgt ttccccctgg aagctccctc     420
   gtgcgctctc ctgttccgac cctgccgctt accggatacc tgtccgcctt tctcccttcg     480
   ggaagcgtgg cgctttctca tagctcacgc tgtaggtatc tcagttcggt gtaggtcgtt     540
   cgctccaagc tgggctgtgt gcacgaaccc cccgttcagc ccgaccgctg cgccttatcc     600
   ggtaactatc gtcttgagtc caacccggta agacacgact tatcgccact ggcagcagcc     660
   actggtaaca ggattagcag agcgaggtat gtaggcggtg ctacagagtt cttgaagtgg     720
   tggcctaact acggctacac tagaaggaca gtatttggta tctgcgctct gctgaagcca     780
   gttaccttcg gaaaaagagt tggtagctct tgatccggca aacaaaccac cgctggtagc     840
   ggtggttttt ttgtttgcaa gcagcagatt acgcgcagaa aaaaaggatc tcaagaagat     900
   cctttgatct tttctacggg gtctgacgct cagtggaacg aaaactcacg tgggcccgat     960
   atcgccgtcc cgtcaagtca gcgtaatgct ctgccagtgt tacaaccaat taaccaattc    1020
   tgattagaaa aactcatcga gcatcaaatg aaactgcaat ttattcatat caggattatc    1080
   aataccatat ttttgaaaaa gccgtttctg taatgaagga gaaaactcac cgaggcagtt    1140
   ccataggatg gcaagatcct ggtatcggtc tgcgattccg actcgtccaa catcaataca    1200
   acctattaat ttcccctcgt caaaaataag gttatcaagt gagaaatcac catgagtgac    1260
   gactgaatcc ggtgagaatg gcaaaagctt atgcatttct ttccagactt gttcaacagg    1320
   ccagccatta cgctcgtcat caaaatcact cgcatcaacc aaaccgttat tcattcgtga    1380
   ttgcgcctga gcgagacgaa atacgcgatc gctgttaaaa ggacaattac aaacaggaat    1440
   cgaatgcaac cggcgcagga acactgccag cgcatcaaca atattttcac ctgaatcagg    1500
   atattcttct aatacctgga atgctgtttt cccggggatc gcagtggtga gtaaccatgc    1560
   atcatcagga gtacggataa aatgcttgat ggtcggaaga ggcataaatt ccgtcagcca    1620
   gtttagtctg accatctcat ctgtaacatc attggcaacg ctacctttgc catgtttcag    1680
   aaacaactct ggcgcatcgg gcttcccata caatcgatag attgtcgcac ctgattgccc 1740
   gacattatcg cgagcccatt tatacccata taaatcagca tccatgttgg aatttaatcg 1800
   cggcctcgag caagacgttt cccgttgaat atggctcata acaccccttg tattactgtt 1860
   tatgtaagca gacagtttta ttgttcatga tgatatattt ttatcttgtg caatgtaaca 1920
   tcagagattt tgagacacac aacgtggcca attgtcagaa ttggttaatt ggttgtaaca 1980
   ctggcagagc attacgctga cttgacggga cggcgatatc gctagcgaca ccatcgaatg 2040
   gcgcaaaacc tttcgcggta tggcatgata gcgcccggaa gagagtcaat tcagggtggt 2100
   gaatgtgaaa ccagtaacgt tatacgatgt cgcagagtat gccggtgtct cttatcagac 2160
   cgtttcccgc gtggtgaacc aggccagcca cgtttctgcg aaaacgcggg aaaaagtgga 2220
   agcggcgatg gcggagctga attacattcc caaccgcgtg gcacaacaac tggcgggcaa 2280
   acagtcgttg ctgattggcg ttgccacctc cagtctggcc ctgcacgcgc cgtcgcaaat 2340
   tgtcgcggcg attaaatctc gcgccgatca actgggtgcc agcgtggtgg tgtcgatggt 2400
   agaacgaagc ggcgtcgaag cctgtaaagc ggcggtgcac aatcttctcg cgcaacgcgt 2460
   cagtgggctg atcattaact atccgctgga tgaccaggat gccattgctg tggaagctgc 2520
   ctgcactaat gttccggcgt tatttcttga tgtctctgac cagacaccca tcaacagtat 2580
   tattttctcc catgaagacg gtacgcgact gggcgtggag catctggtcg cattgggtca 2640
   ccagcaaatc gcgctgttag cgggcccatt aagttctgtc tcggcgcgtc tgcgtctggc 2700
   tggctggcat aaatatctca ctcgcaatca aattcagccg atagcggaac gggaaggcga 2760
   ctggagtgcc atgtccggtt ttcaacaaac catgcaaatg ctgaatgagg gcatcgttcc 2820
   cactgcgatg ctggttgcca acgatcagat ggcgctgggc gcaatgcgcg ccattaccga 2880
   gtccgggctg cgcgttggtg cggatatctc ggtagtggga tacgacgata ccgaagacag 2940
   ctcatgttat atcccgccgt taaccaccat caaacaggat tttcgcctgc tggggcaaac 3000
   cagcgtggac cgcttgctgc aactctctca gggccaggcg gtgaagggca atcagctgtt 3060
   gcccgtctca ctggtgaaaa gaaaaaccac cctggcgccc aatacgcaaa ccgcctctcc 3120
   ccgcgcgttg gccgattcat taatgcagct ggcacgacag gtttcccgac tggaaagcgg 3180
   gcagtgagct agcgggaata agggcgacac ggaaatgttg aatactcata ctcttccttt 3240
   ttcaatatta ttgaagcatt tatcagggtt attgtctcat gagcggatac atatttgaat 3300
   gtatttagaa aaataaacaa ataggggttc cgcgcacatt tccccgaaaa gtgccacctg 3360
   acgtctaaga aaccattatt atcatgacat taacctataa aaataggcgt atcacgaggc 3420
   cctttcgtct cgcgcgtttc ggtgatgacg gtgaaaacct ctgacacatg cagctcccgg 3480
   agacggtcac agcttgtctg taagcggatg ccgggagcag acaagcccgt cagggcgcgt 3540
   cagcgggtgt tggcgggtgt cggggctggc ttaactatgc ggcatcagag cagattgtac 3600
   tgagagtgca ccatatgctc gatggctacg agggcagaca gtaagtggat ttaccataat 3660
   cccttaattg tacgcaccgc taaaacgcgt tcagcgcgat cacggcagca gacaggtaaa 3720
   aatggcaaca aaccacccga aaaactgccg cgatcgcgcc tgataaattt taaccgtatg 3780
   aatacctatg caaccagagg gtacaggcca cattaccccc acttaatcca ctgaagctgc 3840
   catttttcat ggtttcacca tatgcggtgt gaaataccgc acagatgcgt aaggagaaaa 3900
   taccgcatca ggcgccattc gccattcagg ctgcgcaact gttgggaagg gcgatcggtg 3960
   cgggcctctt cgctattacg ccagctggcg aaagggggat gtgctgcaag gcgattaagt 4020
   tgggtaacgc cagggttttc ccagtcacga cgttgtaaaa cgacggccag t 4071
<210> 34
   <211> 50
   <212> DNA
   <213> Artificial Sequence
<220>
   <223> Primer
<400> 34
   aaaggatcca aactcgagag gtacgattta ctaactggaa gaggcactaa 50
<210> 35
   <211> 26
   <212> DNA
   <213> Artificial Sequence
<220>
   <223> Primer
<400> 35
   aaaaagcttt acgcgaacgc gaagtc 26
<210> 36
   <211> 66
   <212> DNA
   <213> Artificial Sequence
<220>
   <223> Primer
<400> 36
   aaaggatcca cagtcctgct aaaacgttcg tttgatatca tttttcctga caccatcgaa 60
   tggcgc 66
<210> 37
   <211> 77
   <212> DNA
   <213> Artificial Sequence
<220>
   <223> Primer
<400> 37
   aaactcgagt tcctgtgtga aattgttatc cgctcacaat tccacacatt atacgagccg       60
   tcactgcccg ctttcca                                                      77
<210> 38
   <211> 30
   <212> DNA
   <213> Artificial Sequence
<220>
   <223> Primer
<400> 38
   aaaaagcttg cgattgtgta ggctggagct 30
<210> 39
   <211> 72
   <212> DNA
   <213> Artificial Sequence
<220>
   <223> Primer
<400> 39
   aaaaagcttt aaaactaacc gggcgatgcg tcaatggcct tgtgaatccc atggtccata       60
   tgaatatcct cc                                                           72
<210> 40
   <211> 73
   <212> DNA
   <213> Artificial Sequence
<220>
   <223> Primer
<400> 40
   aattggatcc acagtcctgc taaaacgttc gtttgatatc atttttcctg acaccatcga       60
   atggcgcaaa acc                                                          73
<210> 41
   <211> 32
   <212> DNA
   <213> Artificial Sequence
<220>
   <223> Primer
<400> 41
   aattcaattg agactcgtgc aactggtcag cg 32
<210> 42
   <211> 9023
   <212> DNA
   <213> Artificial Sequence
<220>
   <223> DNA-Plasmid mit T7-Expressionskonstrukt
<400> 42
   aaaggatcca cagtcctgct aaaacgttcg tttgatatca tttttcctga caccatcgaa      60
   tggcgcaaaa cctttcgcgg tatggcatga tagcgcccgg aagagagtca attcagggtg     120
   gtgaatgtga aaccagtaac gttatacgat gtcgcagagt atgccggtgt ctcttatcag     180
   accgtttccc gcgtggtgaa ccaggccagc cacgtttctg cgaaaacgcg ggaaaaagtg     240
   gaagcggcga tggcggagct gaattacatt cccaaccgcg tggcacaaca actggcgggc     300
   aaacagtcgt tgctgattgg cgttgccacc tccagtctgg ccctgcacgc gccgtcgcaa     360
   attgtcgcgg cgattaaatc tcgcgccgat caactgggtg ccagcgtggt ggtgtcgatg     420
   gtagaacgaa gcggcgtcga agcctgtaaa gcggcggtgc acaatcttct cgcgcaacgc     480
   gtcagtgggc tgatcattaa ctatccgctg gatgaccagg atgccattgc tgtggaagct     540
   gcctgcacta atgttccggc gttatttctt gatgtctctg accagacacc catcaacagt     600
   attattttct cccatgaaga cggtacgcga ctgggcgtgg agcatctggt cgcattgggt     660
   caccagcaaa tcgcgctgtt agcgggccca ttaagttctg tctcggcgcg tctgcgtctg     720
   gctggctggc ataaatatct cactcgcaat caaattcagc cgatagcgga acgggaaggc     780
   gactggagtg ccatgtccgg ttttcaacaa accatgcaaa tgctgaatga gggcatcgtt     840
   cccactgcga tgctggttgc caacgatcag atggcgctgg gcgcaatgcg cgccattacc     900
   gagtccgggc tgcgcgttgg tgcggatatc tcggtagtgg gatacgacga taccgaagac     960
   agctcatgtt atatcccgcc gttaaccacc atcaaacagg attttcgcct gctggggcaa    1020
   accagcgtgg accgcttgct gcaactctct cagggccagg cggtgaaggg caatcagctg    1080
   ttgcccgtct cactggtgaa aagaaaaacc accctggcgc ccaatacgca aaccgcctct    1140
   ccccgcgcgt tggccgattc attaatgcag ctggcacgac aggtttcccg actggaaagc    1200
   gggcagtgag cgcaacgcaa ttaatgtaag ttagctcact cattaggcac cccaggcttt    1260
   acactttatg cttccggctc gtataatgtg tggaattgtg agcggataac aatttcacac    1320
   aggaaacagc tatgaccatg attacggatt cactggccgt cgttttacaa cgtcgtgact    1380
   gggaaaaccc tggcgttacc caacttaatc gccttgcagc acatccccct ttcgccagct 1440
   ggcgtaatag cgaagaggcc cgcaccgatc gcccttccca acagttgcgc agcctgaatg 1500
   gcgaatggcg ctttgcctgg tttccggcac cagaagcggt gccggaaagc tggctggagt 1560
   gcgatcttcc tgaggccgat actgtcgtcg tcccctcaaa ctggcagatg cacggttacg 1620
   atgcgcccat ctacaccaac gtgacctatc ccattacggt caatccgccg tttgttccca 1680
   cggagaatcc gacgggttgt tactcgctca catttaatgt tgatgaaagc tggctacagg 1740
   aaggccagac gcgaattatt tttgatggcg tcgggatctg atccggattt actaactgga 1800
   agaggcacta aatgaacacg attaacatcg ctaagaacga cttctctgac atcgaactgg 1860
   ctgctatccc gttcaacact ctggctgacc attacggtga gcgtttagct cgcgaacagt 1920
   tggcccttga gcatgagtct tacgagatgg gtgaagcacg cttccgcaag atgtttgagc 1980
   gtcaacttaa agctggtgag gttgcggata acgctgccgc caagcctctc atcactaccc 2040
   tactccctaa gatgattgca cgcatcaacg actggtttga ggaagtgaaa gctaagcgcg 2100
   gcaagcgccc gacagccttc cagttcctgc aagaaatcaa gccggaagcc gtagcgtaca 2160
   tcaccattaa gaccactctg gcttgcctaa ccagtgctga caatacaacc gttcaggctg 2220
   tagcaagcgc aatcggtcgg gccattgagg acgaggctcg cttcggtcgt atccgtgacc 2280
   ttgaagctaa gcacttcaag aaaaacgttg aggaacaact caacaagcgc gtagggcacg 2340
   tctacaagaa agcatttatg caagttgtcg aggctgacat gctctctaag ggtctactcg 2400
   gtggcgaggc gtggtcttcg tggcataagg aagactctat tcatgtagga gtacgctgca 2460
   tcgagatgct cattgagtca accggaatgg ttagcttaca ccgccaaaat gctggcgtag 2520
   taggtcaaga ctctgagact atcgaactcg cacctgaata cgctgaggct atcgcaaccc 2580
   gtgcaggtgc gctggctggc atctctccga tgttccaacc ttgcgtagtt cctcctaagc 2640
   cgtggactgg cattactggt ggtggctatt gggctaacgg tcgtcgtcct ctggcgctgg 2700
   tgcgtactca cagtaagaaa gcactgatgc gctacgaaga cgtttacatg cctgaggtgt 2760
   acaaagcgat taacattgcg caaaacaccg catggaaaat caacaagaaa gtcctagcgg 2820
   tcgccaacgt aatcaccaag tggaagcatt gtccggtcga ggacatccct gcgattgagc 2880
   gtgaagaact cccgatgaaa ccggaagaca tcgacatgaa tcctgaggct ctcaccgcgt 2940
   ggaaacgtgc tgccgctgct gtgtaccgca aggacaaggc tcgcaagtct cgccgtatca 3000
   gccttgagtt catgcttgag caagccaata agtttgctaa ccataaggcc atctggttcc 3060
   cttacaacat ggactggcgc ggtcgtgttt acgctgtgtc aatgttcaac ccgcaaggta 3120
   acgatatgac caaaggactg cttacgctgg cgaaaggtaa accaatcggt aaggaaggtt 3180
   actactggct gaaaatccac ggtgcaaact gtgcgggtgt cgataaggtt ccgttccctg 3240
   agcgcatcaa gttcattgag gaaaaccacg agaacatcat ggcttgcgct aagtctccac 3300
   tggagaacac ttggtgggct gagcaagatt ctccgttctg cttccttgcg ttctgctttg 3360
   agtacgctgg ggtacagcac cacggcctga gctataactg ctcccttccg ctggcgtttg 3420
   acgggtcttg ctctggcatc cagcacttct ccgcgatgct ccgagatgag gtaggtggtc 3480
   gcgcggttaa cttgcttcct agtgaaaccg ttcaggacat ctacgggatt gttgctaaga 3540
   aagtcaacga gattctacaa gcagacgcaa tcaatgggac cgataacgaa gtagttaccg 3600
   tgaccgatga gaacactggt gaaatctctg agaaagtcaa gctgggcact aaggcactgg 3660
   ctggtcaatg gctggcttac ggtgttactc gcagtgtgac taagcgttca gtcatgacgc 3720
   tggcttacgg gtccaaagag ttcggcttcc gtcaacaagt gctggaagat accattcagc 3780
   cagctattga ttccggcaag ggtctgatgt tcactcagcc gaatcaggct gctggataca 3840
   tggctaagct gatttgggaa tctgtgagcg tgacggtggt agctgcggtt gaagcaatga 3900
   actggcttaa gtctgctgct aagctgctgg ctgctgaggt caaagataag aagactggag 3960
   agattcttcg caagcgttgc gctgtgcatt gggtaactcc tgatggtttc cctgtgtggc 4020
   aggaatacaa gaagcctatt cagacgcgct tgaacctgat gttcctcggt cagttccgct 4080
   tacagcctac cattaacacc aacaaagata gcgagattga tgcacacaaa caggagtctg 4140
   gtatcgctcc taactttgta cacagccaag acggtagcca ccttcgtaag actgtagtgt 4200
   gggcacacga gaagtacgga atcgaatctt ttgcactgat tcacgactcc ttcggtacca 4260
   ttccggctga cgctgcgaac ctgttcaaag cagtgcgcga aactatggtt gacacatatg 4320
   agtcttgtga tgtactggct gatttctacg accagttcgc tgaccagttg cacgagtctc 4380
   aattggacaa aatgccagca cttccggcta aaggtaactt gaacctccgt gacatcttag 4440
   agtcggactt cgcgttcgcg taaaagcttg cgattgtgta ggctggagct gcttcgaagt 4500
   tcctatactt tctagagaat aggaacttcg gaataggaac ttcaagatcc cctcacgctg 4560
   ccgcaagcac tcagggcgca agggctgcta aaggaagcgg aacacgtaga aagccagtcc 4620
   gcagaaacgg tgctgacccc ggatgaatgt cagctactgg gctatctgga caagggaaaa 4680
   cgcaagcgca aagagaaagc aggtagcttg cagtgggctt acatggcgat agctagactg 4740
   ggcggtttta tggacagcaa gcgaaccgga attgccagct ggggcgccct ctggtaaggt 4800
   tgggaagccc tgcaaagtaa actggatggc tttcttgccg ccaaggatct gatggcgcag 4860
   gggatcaaga tctgatcaag agacaggatg aggatcgttt cgcatgattg aacaagatgg 4920
   attgcacgca ggttctccgg ccgcttgggt ggagaggcta ttcggctatg actgggcaca 4980
   acagacaatc ggctgctctg atgccgccgt gttccggctg tcagcgcagg ggcgcccggt 5040
   tctttttgtc aagaccgacc tgtccggtgc cctgaatgaa ctgcaggacg aggcagcgcg 5100
   gctatcgtgg ctggccacga cgggcgttcc ttgcgcagct gtgctcgacg ttgtcactga 5160
   agcgggaagg gactggctgc tattgggcga agtgccgggg caggatctcc tgtcatctca 5220
   ccttgctcct gccgagaaag tatccatcat ggctgatgca atgcggcggc tgcatacgct 5280
   tgatccggct acctgcccat tcgaccacca agcgaaacat cgcatcgagc gagcacgtac 5340
   tcggatggaa gccggtcttg tcgatcagga tgatctggac gaagagcatc aggggctcgc 5400
   gccagccgaa ctgttcgcca ggctcaaggc gcgcatgccc gacggcgagg atctcgtcgt 5460
   gacccatggc gatgcctgct tgccgaatat catggtggaa aatggccgct tttctggatt 5520
   catcgactgt ggccggctgg gtgtggcgga ccgctatcag gacatagcgt tggctacccg 5580
   tgatattgct gaagagcttg gcggcgaatg ggctgaccgc ttcctcgtgc tttacggtat 5640
   cgccgctccc gattcgcagc gcatcgcctt ctatcgcctt cttgacgagt tcttctgagc 5700
   gggactctgg ggttcgaaat gaccgaccaa gcgacgccca acctgccatc acgagatttc 5760
   gattccaccg ccgccttcta tgaaaggttg ggcttcggaa tcgttttccg ggacgccggc 5820
   tggatgatcc tccagcgcgg ggatctcatg ctggagttct tcgcccaccc cagcttcaaa 5880
   agcgctctga agttcctata ctttctagag aataggaact tcggaatagg aactaaggag 5940
   gatattcata tggaccatgg gattcacaag gccattgacg catcgcccgg ttagttttaa 6000
   agctttttat cactagtgaa ttcgcggccg cctgcaggtc gaccatatgg gagagctccc 6060
   aacgcgttgg atgcatagct tgagtattct atagtgtcac ctaaatagct tggcgtaatc 6120
   atggtcatag ctgtttcctg tgtgaaattg ttatccgctc acaattccac acaacatacg 6180
   agccggaagc ataaagtgta aagcctgggg tgcctaatga gtgagctaac tcacattaat 6240
   tgcgttgcgc tcactgcccg ctttccagtc gggaaacctg tcgtgccagc tgcattaatg 6300
   aatcggccaa cgcgcgggga gaggcggttt gcgtattggg cgctcttccg cttcctcgct 6360
   cactgactcg ctgcgctcgg tcgttcggct gcggcgagcg gtatcagctc actcaaaggc 6420
   ggtaatacgg ttatccacag aatcagggga taacgcagga aagaacatgt gagcaaaagg 6480
   ccagcaaaag gccaggaacc gtaaaaaggc cgcgttgctg gcgtttttcc ataggctccg 6540
   cccccctgac gagcatcaca aaaatcgacg ctcaagtcag aggtggcgaa acccgacagg 6600
   actataaaga taccaggcgt ttccccctgg aagctccctc gtgcgctctc ctgttccgac 6660
   cctgccgctt accggatacc tgtccgcctt tctcccttcg ggaagcgtgg cgctttctca 6720
   tagctcacgc tgtaggtatc tcagttcggt gtaggtcgtt cgctccaagc tgggctgtgt 6780
   gcacgaaccc cccgttcagc ccgaccgctg cgccttatcc ggtaactatc gtcttgagtc 6840
   caacccggta agacacgact tatcgccact ggcagcagcc actggtaaca ggattagcag 6900
   agcgaggtat gtaggcggtg ctacagagtt cttgaagtgg tggcctaact acggctacac 6960
   tagaagaaca gtatttggta tctgcgctct gctgaagcca gttaccttcg gaaaaagagt 7020
   tggtagctct tgatccggca aacaaaccac cgctggtagc ggtggttttt ttgtttgcaa 7080
   gcagcagatt acgcgcagaa aaaaaggatc tcaagaagat cctttgatct tttctacggg 7140
   gtctgacgct cagtggaacg aaaactcacg ttaagggatt ttggtcatga gattatcaaa 7200
   aaggatcttc acctagatcc ttttaaatta aaaatgaagt tttaaatcaa tctaaagtat 7260
   atatgagtaa acttggtctg acagttacca atgcttaatc agtgaggcac ctatctcagc 7320
   gatctgtcta tttcgttcat ccatagttgc ctgactcccc gtcgtgtaga taactacgat 7380
   acgggagggc ttaccatctg gccccagtgc tgcaatgata ccgcgagacc cacgctcacc 7440
   ggctccagat ttatcagcaa taaaccagcc agccggaagg gccgagcgca gaagtggtcc 7500
   tgcaacttta tccgcctcca tccagtctat taattgttgc cgggaagcta gagtaagtag 7560
   ttcgccagtt aatagtttgc gcaacgttgt tgccattgct acaggcatcg tggtgtcacg 7620
   ctcgtcgttt ggtatggctt cattcagctc cggttcccaa cgatcaaggc gagttacatg 7680
   atcccccatg ttgtgcaaaa aagcggttag ctccttcggt cctccgatcg ttgtcagaag 7740
   taagttggcc gcagtgttat cactcatggt tatggcagca ctgcataatt ctcttactgt 7800
   catgccatcc gtaagatgct tttctgtgac tggtgagtac tcaaccaagt cattctgaga 7860
   atagtgtatg cggcgaccga gttgctcttg cccggcgtca atacgggata ataccgcgcc 7920
   acatagcaga actttaaaag tgctcatcat tggaaaacgt tcttcggggc gaaaactctc 7980
   aaggatctta ccgctgttga gatccagttc gatgtaaccc actcgtgcac ccaactgatc 8040
   ttcagcatct tttactttca ccagcgtttc tgggtgagca aaaacaggaa ggcaaaatgc 8100
   cgcaaaaaag ggaataaggg cgacacggaa atgttgaata ctcatactct tcctttttca 8160
   atattattga agcatttatc agggttattg tctcatgagc ggatacatat ttgaatgtat 8220
   ttagaaaaat aaacaaatag gggttccgcg cacatttccc cgaaaagtgc cacctgatgc 8280
   ggtgtgaaat accgcacaga tgcgtaagga gaaaataccg catcaggaaa ttgtaagcgt 8340
   taatattttg ttaaaattcg cgttaaattt ttgttaaatc agctcatttt ttaaccaata 8400
   ggccgaaatc ggcaaaatcc cttataaatc aaaagaatag accgagatag ggttgagtgt 8460
   tgttccagtt tggaacaaga gtccactatt aaagaacgtg gactccaacg tcaaagggcg 8520
   aaaaaccgtc tatcagggcg atggcccact acgtgaacca tcaccctaat caagtttttt 8580
   ggggtcgagg tgccgtaaag cactaaatcg gaaccctaaa gggagccccc gatttagagc 8640
   ttgacgggga aagccggcga acgtggcgag aaaggaaggg aagaaagcga aaggagcggg 8700
   cgctagggcg ctggcaagtg tagcggtcac gctgcgcgta accaccacac ccgccgcgct 8760
   taatgcgccg ctacagggcg cgtccattcg ccattcaggc tgcgcaactg ttgggaaggg 8820
   cgatcggtgc gggcctcttc gctattacgc cagctggcga aagggggatg tgctgcaagg 8880
   cgattaagtt gggtaacgcc agggttttcc cagtcacgac gttgtaaaac gacggccagt 8940
   gaattgtaat acgactcact atagggcgaa ttgggcccga cgtcgcatgc tcccggccgc 9000
   catggcggcc gcgggaattc gat 9023
<210> 43
   <211> 39
   <212> DNA
   <213> Escherichia coli
<400> 43
   acagtcctgc taaaacgttc gtttgatatc atttttcct 39
<210> 44
   <211> 39
   <212> DNA
   <213> Escherichia coli
<400> 44
   gattcacaag gccattgacg catcgcccgg ttagtttta 39